(19) Europäisches Patentamt
European Patent Office
Office européen des brevets

(11) Veröffentlichungsnummer: **0 253 327 B1**

(12) # EUROPÄISCHE PATENTSCHRIFT

(45) Veröffentlichungstag der Patentschrift: **06.05.92**

(51) Int. Cl.[5]: **C07C 211/26**, C07C 217/56, C07D 317/58

(21) Anmeldenummer: **87109989.1**

(22) Anmeldetag: **10.07.87**

Die Akte enthält technische Angaben, die nach dem Eingang der Anmeldung eingereicht wurden und die nicht in dieser Patentschrift enthalten sind.

(54) **Neue Diphenylpropylamin-Derivate, ihre Herstellung sowie ihre pharmazeutische Verwendung.**

(30) Priorität: **11.07.86 HU 287386**
**20.05.87 HU 287386**

(43) Veröffentlichungstag der Anmeldung:
**20.01.88 Patentblatt 88/03**

(45) Bekanntmachung des Hinweises auf die Patenterteilung:
**06.05.92 Patentblatt 92/19**

(84) Benannte Vertragsstaaten:
**AT BE CH DE ES FR GB GR IT LI LU NL SE**

(56) Entgegenhaltungen:
**DE-B- 1 171 930**
**GB-A- 1 032 650**

(73) Patentinhaber: **CHINOIN Gyogyszer és Vegyészeti Termékek Gyára RT.**
**To utca 1-5**
**H-1045 Budapest IV(HU)**

(72) Erfinder: **Korbonits, Dezs , Dr.**
**Vérhalom utca 27/d.**
**H-1025 Budapest(HU)**
Erfinder: **Kiss, Pál, Dr.**
**Vetés utca 82.**
**H-1046 Budapest(HU)**
Erfinder: **Szekeres, Lászl , Dr.**
**Kazinczy utca 2.**
**H-6720 Szeged(HU)**
Erfinder: **Papp, Gyula, Dr.**
**Bécsi krt. 37-39.**
**H-6722 Szeged(HU)**
Erfinder: **Kovács, Gábor, Dr.**
**R na park 4.**
**H-1142 Budapest(HU)**
Erfinder: **Sánta, geb. Csutor, Andrea**
**Frankel Le ut 96.**
**H-1023 Budapest(HU)**
Erfinder: **Virág, Sándor, Dr.**
**Sallai I. u. 29/a.**
**H-1136 Budapest(HU)**
Erfinder: **Udvari, Eva, Dr.**
**Olajos utca 2/a.**
**H-6724 Szeged(HU)**
Erfinder: **Bata, Imre**
**Keleti K. utca 29.**
**H-1024 Budapest(HU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.08/2.19/2.0)

Erfinder: **Mármarosi, geb. Kellner, Katalin**
**Ybl M. sétány 19.**
**H-2051 Biatorbágy(HU)**
Erfinder: **Tardos, Lászl , Dr.**
**Egri I. u. 34.**
**H-1111 Budapest(HU)**
Erfinder: **Körmöczy, Péter, Dr.**
**Uri utca 33.**
**H-1014 Budapest(HU)**
Erfinder: **Gergely, Vera, Dr.**
**Tin di utca 9-11.**
**H-1095 Budapest(HU)**
Erfinder: **Vargai, Zoltán, Dr.**
**Vezér utca 59/b.**
**H-1144 Budapest(HU)**

(74) Vertreter: **Patentanwälte Beetz sen. - Beetz jun. Timpe - Siegfried - Schmitt-Fumian**
**Steinsdorfstrasse 10**
**W-8000 München 22(DE)**

**Beschreibung**

Die Erfindung betrifft neue Diphenylpropylamin-Derivate mit therapeutisch vorteilhaften Eigenschaften, die in erster Linie bei Herz- und Kreislauferkrankungen angewendet werden können, Verfahren zu ihrer Herstellung und entsprechende pharmazeutische Mittel.

Von mehreren Diphenylpropylamin-Derivaten ist bekannt, daß sie vorteilhafte herztherapeutische Eigenschaften besitzen. Hierzu gehört zum Beispiel das Prenylamin (Arzneimittelforschung 10 (1960), 569, 573, 583; Arch. Pharm. 295 (1962), 196). Ein ebenfalls in breitem Maße angewandtes Arzneimittel ist das Fendilin der Formel

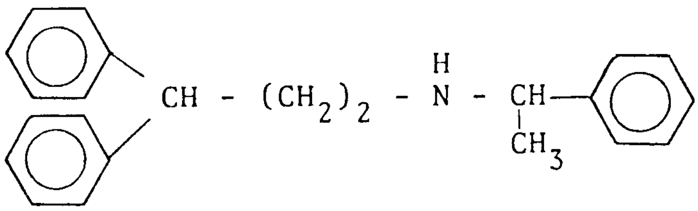

,

das bei ischämischen Herzerkrankungen mit Erweiterung der Coronargefäße und Calciumantagonismus, wie im Falle von Angina pectoris und einigen anderen Kreislauferkrankungen (HU-B-150 534), eingesetzt wird. Es wurden zahlreiche Fendilin-Derivate angegeben, bei denen eine oder beide Phenylgruppen der Diphenylpropylaminogruppe beliebig substituiert sind. Bei keinem dieser Derivate wurde eine bedeutende, das Fendilin übertreffende biologische Wirkung beschrieben (J. für Prakt. Chem. 34 (1966); Magy. Kem. Folyoirat 74 (1968) 20).

Überraschenderweise wurde im Rahmen der Erfindung festgestellt, daß Verbindungen der allgemeinen Formel I vorteilhafte biologische Wirkungen aufweisen:

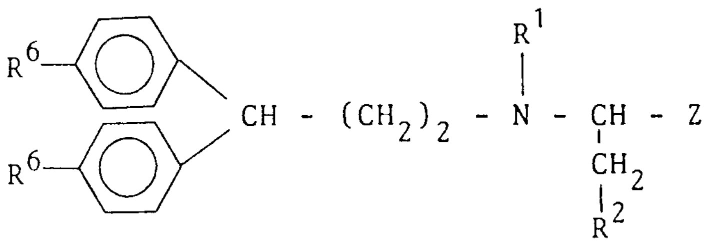

(I),

worin bedeuten:
$R^1$ Wasserstoff oder Methyl,
$R^2$ Wasserstoff, Methyl oder n-Decyl,
Z (a) eine durch $R^3$, $R^4$ und $R^5$ substituierte Phenylgruppe

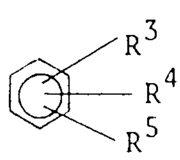

,

wobei bedeuten:
$R^3$ Wasserstoff, Fluor, Chlor oder Brom, Nitro, $C_{1-12}$-Alkyl, $C_{1-4}$-Alkoxy, Phenoxy oder Benzyloxy,
$R^4$ und $R^5$ Wasserstoff, Chlor, Hydroxy, Alkoxy, Benzyloxy, Acetamino, oder Carboxy oder
$R^4$ und $R^5$ zusammen Methylendioxy oder
(b) 4-Methoxynaphthyl oder 4-Ethoxynaphthyl und
$R^6$ Wasserstoff oder Fluor,
und ihre Säureadditionssalze,
wobei solche 2-phenylsubstituierten Verbindungen der Formel I ausgenommen sind, bei denen

(A) $R^1$, $R^2$, $R^3$, $R^4$, $R^5$ und $R^6$ jeweils Wasserstoff
oder
(B) $R^1$, $R^3$, $R^4$, $R^5$ und $R^6$ jeweils Wasserstoff,
und
$R^2$ Methyl
bedeuten.

Die den oben definierten Disclaimern A und B entsprechenden 2-phenylsubstituierten Verbindungen sind aus DE-A-1 171 930 bekannt, wobei auch auf ihre pharmazeutische Brauchbarkeit als Coronardilatantien hingewiesen ist.

Die erfindung betrifft ferner auch die Verbindungen 2-(Diphenyl-4-yl)-6,6-diphenyl-3-azahexan, 2-(3,4-Dimethylphenyl)-6,6-diphenyl-3-azahexan und 2-(2,4-Dimethylphenyl)-6,6-diphenyl-3-azahexan.

Die Derivate der allgemeinen Formel I, bei denen $R^2$ Methyl, $R^3$ Halogen und $R^4$, $R^5$ und $R^6$ Wasserstoff bedeuten, besitzen eine bedeutende antianginöse Wirkung. Das Halogenatom kann dabei Fluor, Chlor oder Brom sein. So ist die antianginöse Wirkung von 2-(4-Chlorphenyl)-6,6-diphenyl-3-azahexan-hydrochlorid bei mit Vasopressin hervorgerufener akuter Coronarinsuffizienz an Ratten außerordentlich stark. Bei intravenöser Verabreichung zwei Minuten vor dem Auslösen der Vasopressin-Angina (2 NE/kg i.v.) beträgt die $ED_{50}$ bei dieser Verbindung 0,054 mg/kg. Im Vergleich hierzu beträgt der gemessene $ED_{50}$-Wert des Fendilins unter ähnlichen Bedingungen 2,30 mg/kg. Die genannte Verbindung ist also unter diesen Bedingungen etwa 42mal wirksamer als Fendilin.

Eine andere, sehr vorteilhafte therapeutische Eigenschaften besitzende Gruppe von Verbindungen der allgemeinen Formel I sind die Derivate, bei denen $R^1$ und $R^2$ Wasserstoff bedeuten und der Benzolring der Phenylethylgruppe mehrfach substituiert ist.

In diesem Fall ist besonders vorteilhaft, wenn der Substituent $R^3$ eine Alkoxygruppe ist. Verbindungen dieses Typs haben nicht nur die für das Fendilin charakteristische, aber stärkere und länger andauernde antianginöse Wirkung, sondern sie besitzen überraschenderweise auch in der Therapie von Herzerkrankungen vorteilhafte andere, neuartige Wirkungen. So übertrifft das 2-(3,4-Dimethoxyphenyl)-6,6-diphenyl-3-azahexan (das Hydrochlorid dieser Verbindung ist im folgenden als KHL-8430 abgekürzt) bei Vasopressin-Angina an Ratten beziehungsweise bei durch Coronarocclusion ausgelösten ischämischen Zuständen an Hunden das Fendilin in Stärke und Dauer der pharmakologischen Wirkung bei weitem, außerdem ist es bei i.v.- und p.o.-Verabreichung auch weniger toxisch als diese Vergleichsverbindung.

In Tabelle 1 sind für Vasopressin-Angina bei Ratten (Papp und Szekeres, Arch. Int. Pharmacodyn. 160 - (1966) 147) 2 Minuten nach der i.v.-Verabreichung beziehungsweise 60 Minuten nach der p.o.-Verabreichung gemessene antianginöse Aktivität, die an den Ratten gemessenen i.v.- und p.o.-Werte der akuten Toxizität, die therapeutischen Indices und der relative therapeutische Index für KHL-8430 und Fendilin aufgeführt.

Tabelle 1

| Verbindung | i.v. | | | | oral | | | |
|---|---|---|---|---|---|---|---|---|
| | $ED_{50}$ mg/kg | $LD_{50}$ mg/kg | $\frac{LD_{50}}{ED_{50}}$ | Rel. Index* | $ED_{50}$ mg/kg | $LD_{50}$ mg/kg | $\frac{LD_{50}}{ED_{50}}$ | Rel. Index* |
| KHL-8430 | 0,34 | 16,0 | 47,1 | 8,89 | 8,4 | 1400 | 166,7 | 4,17 |
| Fendilin | 2,30 | 12,2 | 5,3 | | 20,0 | 800 | 40,0 | |

$$* = \frac{\text{KHL-8430}}{\text{Fendilin}}$$

Das KHL-8430 senkt an Hunden die durch Coronarocclusion ausgelöste Myocardialischämie (Szekeres und Mitarb., J.Pharm. Exp. Ther. 196 (1976) 15) bei i.v.- und p.o.-Verabreichung ebenfalls stärker und hauptsächlich erheblich länger als Fendilin. Den detaillierten hämodynamischen Untersuchungen an Hunden zufolge senkt das KHL-8430 gegenüber Fendilin und anderen bekannten Calciumantagonisten (zum Beispiel Verapamil, Nifedipin) den arteriellen Blutdruck unter Coronarocclusion nur in geringem Maße, es senkt die Herzfrequenz nicht und verringert an den ischämischen Gebieten des Herzens die Ausdehnung der Aktivierungszeit bedeutend, was ein wichtiger Faktor für die Ausbildung von Arrhythmien des sogenannten "Re-entry"-Typs ist.

Mit KHL-8430 können als im Gegensatz zu Fendilin überraschenderweise die durch Coronarocclusion entstehenden Kammer-Extrasystolen vermindert werden. Seine antiarrhythmische Wirkung ist so bedeutend, daß es bei der Vorkammer-und Kammermuskulatur von Katzenherzen in situ eine Fendilin, Verapamil und andere Calciumantagonisten bei weitem übertreffende und mit einem in der Therapie in breitem Maße angewandten Arzneimittel, dem Mexiletin, identische Wirkung aufweist. An wachen Hunden senkt das KHL-8430 in einer i.v.-Dosis von 2 mg/kg die 24 Stunden nach der Harris'schen "zweistufigen" Coronarabbindung auftretende extrasystolische Aktivität bedeutend, während Fendilin und Verapamil an diesen Arrhythmie-Modellen keine signifikante Wirkung zeigen. Bei oraler Verabreichung senkt KHL-8430 auch die Häufigkeit der Extrasystolen in sehr bedeutendem Maße und in Abhängigkeit von der Dosis. Die Tatsache, daß es bei einem Großteil der Fälle die Herzrhythmusstörungen auch in vollem Umfang aufheben konnte, weist auf die sehr hohe Wirksamkeit des KHL-8430 hin.

Ein spezieller Vorteil besteht auch darin, daß bei oraler Gabe von KHL-8430 die starke antiischämische Schutzwirkung sehr gleichmäßig ist, was in therapeutischer Hinsicht das Einstellen des gewünschten konstanten Blutspiegels außerordentlich begünstigt.

In diesen sehr günstigen Wirkungen bei der Therapie von Herzerkrankungen ist das KHL-8430 aufgrund der detaillierten Untersuchungen des Mechanismus organspezifisch; hierfür können vor allem die in den Coronarien zur Geltung kommende calciumantagonistische Wirkung beziehungsweise auch die schnelle Blockierung des Natriumkanals verantwortlich gemacht werden. Die in diesen engeren Kreis der Verbindungen der allgemeinen Formel I gehörenden Verbindungen besitzen im Gegensatz zu Fendilin und anderen Calciumantagonisten im antianginösen bzw. antiarrhythmischen Dosisbereich keine cardiodepressive Wirkung, was ein herausragender Vorteil ist.

Bei den Untersuchungen ergab sich beim Vergleich der hämodynamisch wirksamen (den Blutdruck beziehungsweise die Kontraktilität der linken Kammer senkenden) sowie der antiischämischen ("antianginösen") und antiarrhythmischen ("antifibrillären") Dosen ($ED_{25}$) von i.v. verabreichtem KHL-8430,

Verapamil und Fendilin, daß der "hämodynamische therapeutische Index" der untersuchten drei Calciumantagonisten bei KHL-8430 bei weitem am günstigsten ist, wie aus Tabelle 1 hervorgeht.

Tabelle 1

Antiischämische, antiarrhythmische, hämodynamische Wirkung des Calciumantagonisten

| Parameter und Tendenz der Veränderung | Verapamil | Fendilin | KHL-8430 |
|---|---|---|---|
| Hämodynamische Angaben: | | | |
| BP | 0,20 | 0,74 | 2,25 |
| HR | 0,75 | 5,00 | 7,20 |
| dP/dt max | 0,07 | 0,49 | 6,00 |
| FFT der Vorkammer | 1,5 | 2,8 | 1,8 |
| durch Vasopressin ausgelöste Angina | 0,1 | 1,2 | 0,6 |
| $\frac{\text{BP}}{\text{antianginös}}$ $(ED_{25})$ | 2,0 | 0,62 | 3,75 |
| $\frac{\text{BP}}{\text{FFT der Vorkammer}}$ $(ED_{25})$ | 0,13 | 0,26 | 1,25 |
| $\frac{\text{dP/dt max}}{\text{antianginös}}$ $(ED_{25})$ | 0,7 | 0,41 | 10,00 |
| $\frac{\text{dP/dt max}}{\text{FFT der Vorkammer}}$ $(ED_{25})$ | 0,04 | 0,175 | 3,33 |

Anmerkung: BP = Blutdruck
HR - Herzfrequenz
FFT = Fibrillationsschwelle
dP/dt max = Kontraktilität der linken Kammer.

Die Verfahren zur Herstellung der erfindungsgemäßen Verbindungen sind durch folgende Maßnahmen gekennzeichnet:

(A) Zur Herstellung von Verbindungen der allgemeinen Formel I mit $R^1$ = H:

(A1) an sich bekannte einstufige oder zweistufige reduktive Kondensation eines Diphenylpropylamins der Formel II

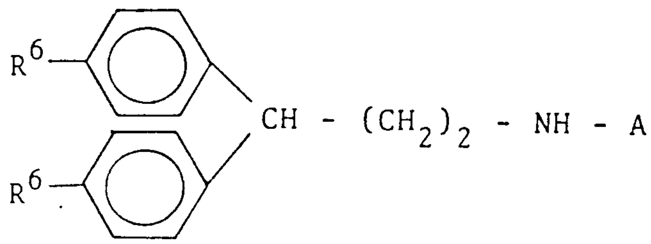 (II),

worin A Wasserstoff und $R^6$ Wasserstoff oder Fluor bedeuten, mit einem Keton der allgemeinen Formel III

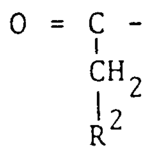 (III),

mit $R^2$ und Z wie oben;
oder
(A2) Umsetzung einer Verbindung der allgemeinen Formel IV

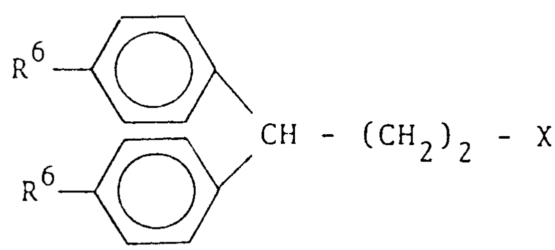 (IV),

worin X Halogen, vorzugsweise Chlor, Brom oder Jod,
und $R^6$ Wasserstoff oder Fluor bedeuten, mit einem Amin der allgemeinen Formel V

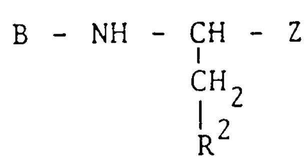 (V),

worin B Wasserstoff oder Benzyl und $R^2$ und Z dasselbe wie oben bedeuten,
und
Debenzylierung der erhaltenen Verbindung, wenn B Benzyl ist;
oder

(A3) Umsetzung eines Amins der allgemeinen Formel II, worin A Wasserstoff oder Benzyl und $R^6$ Wasserstoff oder Fluor bedeuten, mit einer Verbindung der allgemeinen Formel VI

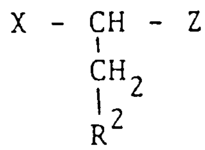

(VI),

worin $R^2$, Z und X die oben angegebene Bedeutung besitzen,
und
Debenzylierung der erhaltenen Verbindung, wenn A Benzyl ist;
oder
(A4) an sich bekannte einstufige oder zweistufige reduktive Kondensation eines Diphenylpropionaldehyds der Formel VII

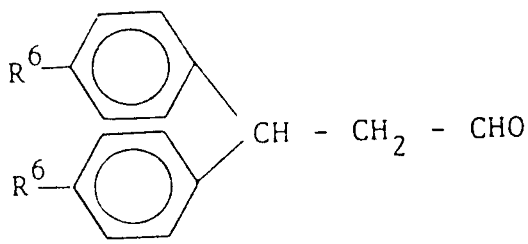

(VII),

worin $R^6$ Wasserstoff oder Fluor bedeutet,
mit einem Amin der Formel V, worin B Wasserstoff und $R^2$ und Z dasselbe wie oben bedeuten;
oder
(A5) an sich bekannte Umsetzung eines Diphenylacetonitrils der Formel VIII

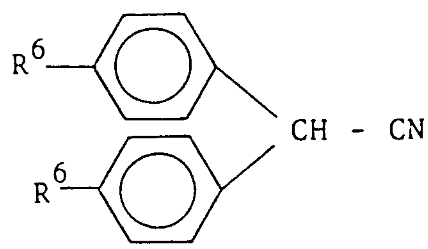

(VIII),

worin $R^6$ Wasserstoff oder Fluor bedeutet, mit einer Verbindung der allgemeinen Formel IX

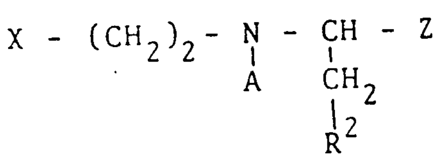

(IX),

worin $R^2$, Z und X die oben angegebene Bedeutung besitzen und A Benzyl ist,
und
Austausch der Cyanogruppe und der Benzylgruppe gegen Wasserstoff bei der so erhaltenen Verbindung;
oder

(A6) Umsetzung eines Amins der allgemeinen Formel X

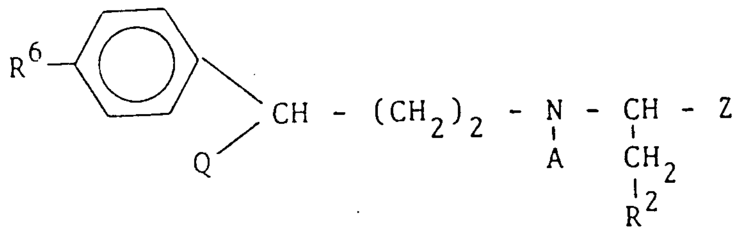

(X),

worin Q Hydroxy oder Halogen, A Wasserstoff oder Benzyl, $R^2$, $R^6$ und Z dasselbe wie oben bedeuten,
oder eines Salzes davon in einer Friedel-Crafts-Reaktion mit Benzol oder Fluorbenzol
und
Debenzylierung der erhaltenen Verbindung, wenn A Benzyl ist;
oder

(B) zur Herstellung von Verbindungen der allgemeinen Formel I mit $R^1$ = Methyl;

(B1) N-Methylierung einer Verbindung der allgemeinen Formel I mit $R^1$ = H in an sich bekannter Weise unter Überführung in ein tertiäres Amin,
oder

(B2) Umsetzung eines Amins der Formel XI

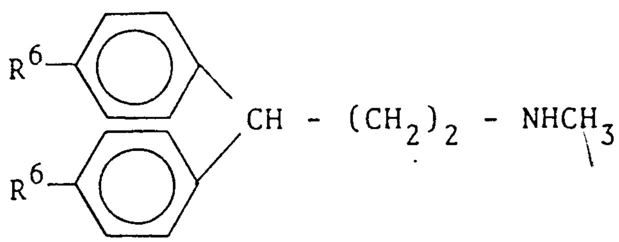

(XI),

worin $R^6$ Wasserstoff oder Fluor bedeutet,
mit einer Verbindung der allgemeinen Formel VI, worin $R^2$, X und Z die oben angegebene Bedeutung besitzen;
oder

(B3) Umsetzung eines Amins der allgemeinen Formel XII

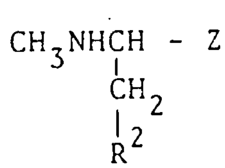

(XII),

worin Z und $R^2$ dasselbe wie oben bedeuten, mit einer Verbindung der allgemeinen Formel IV, worin $R^6$ und X die beim Verfahren A2 angegebene Bedeutung besitzen,
und gewünschtenfalls Überführung der jeweils erhaltenen Verbindungen der Formel I mit anorganischen oder organischen Säuren in entsprechende Säureadditionssalze, insbesondere physiologisch gut verträgliche Salze.

Das Verfahren zur Herstellung der Verbindungen 2-(Diphenyl-4-yl)-6,6-diphenyl-3-azahexan, 2-(3,4-Dimethylphenyl)-6,6-diphenyl-3-azahexan und 2-(2,4-Dimethylphenyl)-6,6-diphenyl-3-azahexan ist gemäß der Erfindung gekennzeichnet durch

(A1) an sich bekannte einstufige oder zweistufige reduktive Kondensation von 3,3-Diphenylpropylamin der Formel IIa

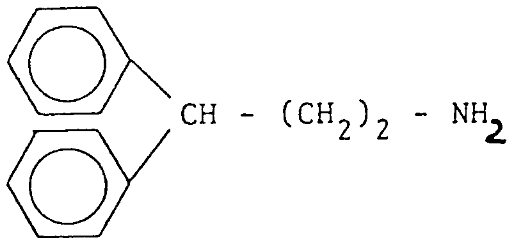

(IIa)

mit einem Keton der allgemeinen Formel IIIa,

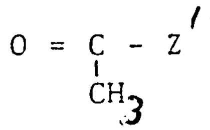

(IIIa),

in der Z' Diphenyl-4-yl, 3,4-Dimethylphenyl oder 2,4-Dimethylphenyl bedeutet, und gewünschtenfalls Überführung der jeweils erhaltenen Verbindung mit anorganischen oder organischen Säuren in ein entsprechendes Säureadditionssalz.

Bei den Verfahren A1 und A4 kann die reduktive Kondensation in zwei Schritten durchgeführt werden, indem durch Umsetzen der primären Amine der Formeln II, IIa beziehungsweise V mit den Carbonylverbindungen der Formeln III, IIIa beziehungsweise VII die Schiff'schen Basen Z.B. der Formel XIII

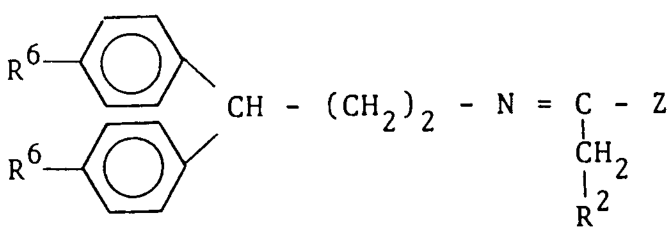

(XIII)

beziehungsweise XIV

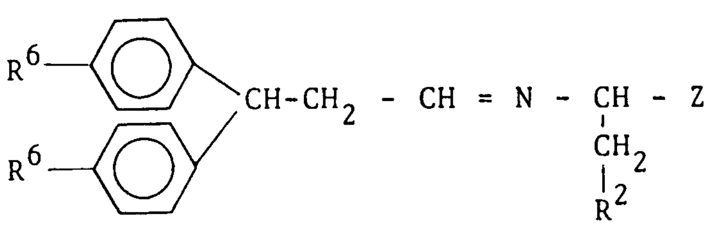

(XIV)

hergestellt und diese dann zu den Zielverbindungen reduziert werden. Die Kondensation kann in einem organischen Lösungsmittel, zum Beispiel in Benzol, Toluol und/oder Xylol, durchgeführt werden. Zur Beschleunigung der Reaktion kann dabei das entstehende Wasser abgetrennt werden, z. B. mit einem wasserabscheidenden Aufsatz. Die Kondensation kann auch in einem lösungsmittelfreien Medium durch Vermischen der Amin- und der Carbonylverbindungen vorgenommen werden, wobei die Wasserabspaltung durch Erwärmen und Anwendung von vermindertem Druck erheblich begünstigt wird.

Die Reduktion der Schiff'schen Base erfolgt vorzugsweise durch katalytische Hydrierung mit üblichen Metall- und Edelmetallkatalysatoren, zum Beispiel mit Nickel-, Palladium- und Platinkatalysatoren. Die Katalyse kann bei Atmosphärendruck oder Wasserstoffüberdruck bei einer Temperatur von 20 - 120° C, vorzugsweise bei 18 - 30° C, und einem Druck von 10000 - 100000 Pa, durchgeführt werden. Als Lösungsmittel können vorzugsweise Alkohole (zum Beispiel Methanol oder Ethanol) sowie Dioxan verwendet werden. Die Reaktion kann auch mit chemischen Reduktionsmitteln, zum Beispiel mit komplexen Metallhydriden, vorzugsweise mit Natriumtetrahydroborat, oder mit Aluminiumamalgam oder Natriumamalgam oder durch elektrolytische Reduktion durchgeführt werden.

Die reduktive Kondensation kann auch in zwei Stufen vorgenommen werden, wobei als Lösungsmittel vorzugsweise Ethanol und als Reduktionsmittel Palladiumkohle verwendet werden.

Bei den Verfahren A2, A3, B2 und B3 können als Lösungsmittel Diethylether, Dichlormethan, Chloroform, Benzol, Aceton und/oder Alkohole sowie sehr vorteilhaft Dimethylformamid verwendet werden. Das Verbinden kann durch Erhitzen auf 50 - 150° C beschleunigt werden. Die bei der Reaktion entstehende Halogenwasserstoffsäure kann mit üblichen anorganischen Basen, vorzugsweise mit Kaliumcarbonat, gebunden werden, die Reaktion kann aber auch in Gegenwart von organischen tertiären Basen oder in Gegenwart von im Überschuß vorhandenem reagierenden Amin durchgeführt werden.

Beim Verfahren A5 kann die Reaktion in Gegenwart üblicher Säurebindungsmittel vorgenommen werden, wobei im ersten Schritt eine durch eine Cyanogruppe substituierte Verbindung der allgemeinen Formel I entsteht, bei der die Cyanogruppe in an sich bekannter Weise gegen ein Wasserstoffatom ausgetauscht werden kann. Das Verfahren wird vorzugsweise so durchgeführt, daß in einem inerten Lösungsmittel, zum Beispiel Toluol oder Benzol, Natriumamid als Säurebindungsmittel und als die Cyanogruppe entfernendes Reagens verwendet wird.

Das Verfahren A6 kann unter Anwendung der in DD-A-33 285 beschriebenen Methoden durchgeführt werden. Die Reaktion findet mit einem Friedel-Crafts-Katalysator, vorzugsweise mit Aluminiumchlorid, in Benzol bei der Siedetemperatur des Mediums statt, wobei das Benzol auch als Reagens dient. Dabei kann auch von einem 1-Phenyl-1,2-alkenylamin-Derivat ausgegangen werden, das vorzugsweise aus der entsprechenden Verbindung der allgemeinen Formel X durch Wasserabspaltung oder Abspaltung der Halogenwasserstoffsäure hergestellt werden kann.

Die als Ausgangsstoffe dienenden Verbindungen der allgemeinen Formel X können hergestellt werden, indem man 3-Phenyl-3-hydroxypropylamin mit dem entsprechenden Keton der Formel III einer reduktiven Kondensation unterwirft und dann gewünschtenfalls die Hydroxylgruppe mit Thionylchlorid gegen ein Chloratom austauscht; der Austausch der Hydroxylgruppe kann aber auch mit der in der Friedel-Crafts-Reaktion entstehenden Halogenwasserstoffsäure vorgenommen werden.

Die Schutzgruppen A und B können während der obigen Reaktionen durch katalytische Hydrierung in Essigsäure unter Verwendung von Platinoxid- oder Palladiumkatalysatoren mit einer annähernd theoretischen Ausbeute entfernt werden.

Beim Verfahren B1 werden vorzugsweise die Verbindungen der allgemeinen Formel I, worin $R^1$ ein Wasserstoffatom ist, in Ameisensäure-Lösung durch Erhitzen mit wässeriger Formaldehydlösung in entsprechende tertiäre Amine übergeführt.

Die Verfahren B2 und B3 können vorteilhaft gemäß den Verfahren A2 und A3 verwirklicht werden.

Die Verbindungen der allgemeinen Formel I werden mit organischen oder anorganischen Säuren in ihre entsprechenden Salze übergeführt. Als organische und anorganische salzbildende Stoffe können vorteilhaft Salzsäure, Schwefelsäure, Phosphorsäure, Milchsäure, Weinsäure, Citronensäure, Maleinsäure und/oder Nicotinsäure verwendet werden. Die Verbindungen der allgemeinen Formel I und ihre physiologisch verträglichen Salze können mit neutralen, nicht toxischen, anorganischen und/oder organischen Trägerstoffen und in der Arzneimittelindustrie gebräuchlichen anderen Hilfsstoffen zu Arzneimittelpräparaten formuliert werden. Solche Arzneimittelpräparate können Tabletten, Filmtabletten, Dragees, dünndarmlösliche Dragees, Zäpfchen, Kapseln, Mikrokapseln, flüssige oder feste Suspensionen, Emulsionen oder Lösungen sein. Als Trägerstoff können vorzugsweise Talkum, verschiedene Dextrinderivate, Gelatine, Wasser und/oder poly(alkylen-alkohol)e dienen. Die Präparate können auch mit weiteren Hilfsstoffen, wie beispielsweise mit Emulgier- bzw. Suspendiermitteln, Salzen, Puffersubstanzen, den Zerfall begünstigenden und anderen therapeutisch wirksamen Stoffen hergestellt werden.

Die erfindungsgemäßen Verbindungen können in Abhängigkeit von der Anwendungsart, vom Zweck, von der Körpermasse sowie vom Alter des Patienten und von anderen Parametern in Dosen von 1 - 300 mg angewandt werden.

Die Erfindung wird im folgenden anhand von Beispielen näher erläutert.

Beispiel 1

Herstellung von 2-(3,4-Dimethylphenyl)-6,6-diphenyl-3-azahexan der Formel

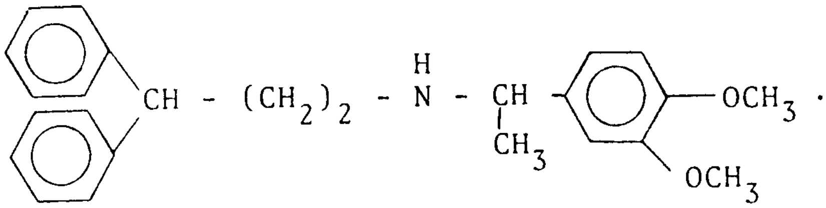

Das Hydrochlorid ist im folgenden mit KHL-8430 abgekürzt.

(a1) Ein Gemisch aus 3,3-Diphenylpropylamin (84,5 g; 0,4 mol) und 3,4-Dimethoxyacetophenon (72,1 g; 0,4 mol) wird unter Rühren bei einem Druck von 130 - 160 Pa 10 Stunden lang auf 90 °C gehalten, wobei das entstehende Wasser ständig abdestilliert wird. Die Masse des nach dem Abkühlen fest werdenden rohen N-(α-Methyl-3,4-dimethoxy-benzyliden)-3,3-diphenylpropylamins beträgt 149,5 g (theoretische Ausbeute). Der Schmelzpunkt liegt bei 97 - 102 °C und erhöht sich durch Kristallisieren aus Ethanol auf 105 - 106 °C.

(a2) Zu 1000 ml einer Suspension der gemäß (a1) hergestellten rohen Schiff'schen-Base in Methanol wird unter Rühren innerhalb von 40 min Natriumtetrahydroborat (37,8 g; 1,0 mol) gegeben; dann wird das Gemisch 3 Stunden lang gerührt. Danach wird das Lösungsmittel unter vermindertem Druck abdestilliert; zum Rückstand wird 800 ml Wasser gegeben, worauf dreimal mit 200 ml Chloroform extrahiert wird. Die vereinigten organischen Phasen werden eingedampft; durch Ansäuern mit salzsaurem Ethylacetat bis zu pH 1 wird das Hydrochlorid gebildet. Ausbeute 145,0 g (88 %); F. 171 - 173 °C (aus wässerigem Ethanol).

(b) Ein Gemisch aus der gemäß (a1) hergestellten umkristallisierten Schiff'schen Base (112,04 g; 0,3 mol), 500 ml Ethanol und 16 g 10 %-iger Palladium/Aktivkohle wird bei 25 °C und Atmosphärendruck bis zur Beendigung der Wasserstoffaufnahme hydriert. Nach Filtrieren und Eindampfen wird der Rest mit 30 ml Salzsäure (37 %-ig) bis zu pH 1 angesäuert; dann wird das entstandene Salz mit 140 ml Wasser vermischt. So erhält man 119 g (96,3 %) KHL-8430; F. 171 - 173 °C.

(c) Ein Gemisch aus 3,3-Diphenylpropylamin (21,1 g; 0,1 mol), 3,4-Dimethoxyacetophenon (18,0 g; 0,1 mol) und 200 ml Xylol wird mit einem Wasserabscheider-Aufsatz bis zur Beendigung der Wasserabtrennung erhitzt. Das Lösungsmittel wird dann bei vermindertem Druck abdestilliert; die zurückgebliebene Schiff'sche Base (75 g) wird mit einem Gemisch aus 250 ml Ethanol und 2 g 10 %-iger Palladium/Aktivkohle gemäß (b) hydriert und aufgearbeitet. Man erhält 31,5 g (84 %) KHL-8430.

(d) Ein Gemisch aus 3,3-Diphenylpropylamin (21,1 g; 0,1 mol), 3,4-Dimethoxyacetophenon (18,0 g; 0,1 mol), 200 ml Ethanol und 2 g 10 %-iger Palladium/Aktivkohle wird bei einem Druck von 1,0 MPa hydriert. Nach Filtrieren, Eindampfen, Salzbildung mit salzsaurem Ethylacetat und Kristallisieren aus Ethanol erhält man 27,2 g (73 %) KHL-8430; F. 171 - 173 °C.

(e) Aus der gemäß (a2) hergestellten 2-(3,4-Dimethoxyphenyl)-6,6-diphenyl-3-azahexan-Base wird in Ethanol-Medium mit 1 Äquivalent 2 N Schwefelsäure das Sulfat hergestellt; F. 195 - 198 °C.

(f) Ein Gemisch aus der gemäß Beispiel (a1) hergestellten rohren Schiff'schen Base, 1000 ml Ethanol und 4 g Raney-Nickel wird bei Atmosphärendruck und 20 °C hydriert. Nach Filtrieren und Eindampfen wird mit salzsaurem Ethylacetat oder salzsaurem Ethanol das Hydrochlorid gebildet Man erhält 145,0g- (88 % ) KHL-8430; F. 171 - 173 °C.

(g) In analoger Weise wie bei (e) sind das Hydrobromid (F. 176 - 178 °C), das Nitrat (F. 150 - 152 °C) und das Nicotinat (F. 105 - 106 °C) zugänglich.

Beispiel 2

(a) Ein Gemisch aus 28,0 g 1-Brom-3,3-diphenylpropan, 27,1 g 1-(3,4-Dimethoxyphenyl)-1-benzylaminomethan, 15,0 g Kaliumcarbonat und 100 ml Dimethylformamid wird 16 h lang bei 70 °C gerührt. Nach dem Filtrieren wird das Lösungsmittel unter vermindertem Druck abdestilliert; der Rückstand wird in 100 ml Eisessiglösung mit 0,5 g Platinoxid-Katalysator bei 70 °C und 0,4 MPa hydriert. Nach Beendigung der Wasserstoffaufnahme (etwa 5 h) wird mit 100 ml Methanol verdünnt, filtriert und das Lösungsmittel

abdestilliert. Aus dem Rückstand wird gemäß Beispiel 1 (a2) das Hydrochlorid gebildet und aus Ethanol kristallisiert. Man erhält 31,0 g KHL-8430 (F. 171-173°C), das mit der gemäß Beispiel 1 (a2) hergestellten Verbindung identisch ist.

Beispiel 3

(a) Ein Gemisch aus 18,1 g 1-(3,4-Dimethoxyphenyl)-1-aminoethan (J. Chem. Soc. 1963, 4289), 10,6 g Benzaldehyd, 100 ml Methanol und 1 Tropfen Pyridin läßt man bei 20°C zwei Tage lang stehen; dann werden innerhalb von 30 min 3,8 g Natriumtetrahydroborat zu dem Gemisch zugegeben, worauf 3 h lang gerührt wird. Das Lösungsmittel wird dann abdestilliert; der Rückstand wird mit Wasser verdünnt und mit Chloroform extrahiert. Nach dem Trocknen wird das Chloroform abgedampft. Das so gewonnene 1-Phenyl-3-(3,4-dimethoxyphenyl)-2-azabutan (27,1 g) kann unmittelbar weiterverarbeitet werden.

(b) Die in (a) erhaltenen 27,1 g 1-Phenyl-3-(3,4-dimethoxyphenyl)-2-azabutan werden in 80 ml Dimethylformamid bei 70°C unter Rühren innerhalb 1 h tropfenweise mit 7,1 g 2-Chlorethanol versetzt. Dann wird noch weitere 5 h gerührt und danach filtriert. Das Filtrat wird eingedampft, in 150 ml Chloroform aufgelöst und mit 20 ml Thionylchlorid 1 h lang erhitzt (bis zum Aufhören der Gasentwicklung). Das nach dem Eindampfen zurückgebliebene Salz wird in Wasser gelöst, unter Eiskühlung alkalisch gemacht und mit Ether extrahiert. Die Etherlösung wird über Natriumsulfat getrocknet und filtriert. Nach Abdestillieren des Ethers bleibt das entstandene 1-[N-[1-(3,4-Dimethoxypheny)-ethan-1-yl]-N-benzyl]-amino-2-chorethan als Öl zurück.

Von diesem Rohprodukt werden 16,5 g in 100 ml Benzol gelöst und unter Rühren mit 9,7g Diphenylacetonitril und 2,5 g Natriumamid 2 h lang erhitzt. Nach dem Abkühlen wird Wasser zugemischt, die Benzolphase abgetrennt, filtriert und eingedampft. Der Rückstand wird in Diethylether gelöst; das mit salzsaurem Ethanol gebildete 1,1-Diphenyl-1-cyano-3-[N-[1-(3,4-dimethoxyphenyl)-ethan-1-yl]-N-benzyl]-aminopropan-hydrochlorid wird aus Ethanol kristallisiert.

Von dem so erhaltenen Hydrochlorid werden 10 g in 100 ml Wasser gelöst, worauf unter Kühlen alkalisch gemacht, mit 100 ml Benzol extrahiert, über Natriumsulfat getrocknet und filtriert wird. Das Filtrat wird mit 10,0 g Natriumamid 2 h unter Rühren erhitzt, nach dem Abkühlen filtriert und eingedampft. Der Rückstand wird in 50 ml konzentrierter Essigsäure aufgelöst und mit 0,1 g Platinoxid-Katalysator bei 70 °C und 0,4 MPa etwa 5 h hydriert. Das Gemisch wird dank mit 50 ml Methanol verdünnt, abfiltriert und eingedampft; durch leichtes Erhitzen mit salzsaurem Ethanol wird das Salz gebildet. Nach Kristallisieren mit wässerigem Ethanol erhält man 6,2 g KHL-8430; F. 171-172 °C.

Beispiel 4

Zu einem Gemisch aus 18,1 g 1-(3,4-Dimethoxyphenyl)-1-aminoethan, 15 g Kaliumcarbonat und 100 ml Butanol werden unter Rühren und Erhitzen innerhalb von etwa 1 h 50 ml einer Lösung von 23,7 g 1-Chlor-3,3-diphenylpropan in Butanol gegeben, worauf bis zur Beendigung der Gasentwicklung weiter erhitzt wird. Nach dem Abkühlen wird filtriert und eingedampft; gemäß Beispiel 1 (a2) wird das Hydrochlorid erzeugt und aus wässerigem Ethanol zweimal kristallisiert. Man erhält 28,5 g KHL-8430; F. 171-172°C.

Beispiel 5

Ein Gemisch aus 21,0 g 1-Amino-3,3-diphenylpropan, 24,5 g 1-Brom-1-(3,4-dimethoxyphenyl)-ethan (Bull. Soc. Chim. France, 1973, 2665), 15 g Kaliumcarbonat und 80 ml Dimethylformamid wird bei 50°C 15 h lang gerührt und filtriert. Dann wird das Lösungsmittel unter vermindertem Druck abdestilliert und der Rückstand in Chloroform aufgenommen. Nach Salzbildung gemäß Beispiel 1 (a2) und zweimaligem Kristallisieren aus wässerigem Ethanol erhält man 24,0 g KHL-8430; F. 171 - 173°C.

Beispiel 6

180 ml einer ethanolischen Lösung von 21,0 g 3,3-Diphenylpropionaldehyd (J. Med. Chem. 7 (1964), 623) und 18,1 g 1-(3,4-Dimethoxyphenyl)-1-aminoethan wird 40 min lang erhitzt; dann wird das Gemisch nach Zugabe von 2 ml Wasser bei 33 - 35°C innerhalb von 30 min mit 4 g Natriumtetrahydroborat umgesetzt. Nach Abdestillieren des Alkohols wird der Rückstand mit Wasser behandelt und mit Chloroform extrahiert; die Chloroformlösung wird gemäß Beispiel 1 (a2) aufgearbeitet. Man erhält 30,0 g KHL-8430; F. 171 - 173°C (aus wässerigem Ethanol).

Beispiel 7

15,1 g 1-Phenyl-1-hydroxy-3-aminopropan und 19,0 g 3,4-Dimethoxyacetophenon werden bei 80 °C und 130-160 Pa 16 h lang gerührt. Nach dem Abkühlen wird ein Gemisch von 50 ml Methanol und 2 ml Wasser zugemischt und mit 3,5 g Natriumtetrahydroborat bei 30-40 °C 2 h lang umgesetzt. Nach Abdestillieren des Lösungsmittels wird der Rückstand in 100 ml Ether aufgenommen; mit salzsaurem Ethanol wird das Salz gebildet. Das so erhaltene rohe 1-Phenyl-1-hydroxy-3-[N-[1-(3,4-dimethoxyphenyl)-ethan-1-yl]]-aminopropan-hydrochlorid wird in 100 ml Benzol suspendiert; dann werden unter Rühren innerhalb von 40 min 40 ml Thionychlorid zugegeben; die Suspension wird noch eine weitere Stunde lang bei 40 °C gerührt. Das Gemisch wird danach im Wasserstrahlpumpenrahmen eingedampft (max. 40 °C); der Rückstand wird in 100 ml Benzol aufgenommen und mit 20 g Aluminiumchlorid bei 55-60 °C 1 h lang und dann unter Erhitzen weitere 2 h umgesetzt. Nach dem Abkühlen wird das Gemisch auf Salzsäure enthaltendes Eis gegossen. Die Benzolphase wird abgetrennt und das Benzol mit Wasser ausgewaschen. Die vereinigten wässerigen Phasen werden unter Eiskühlung stark alkalisch gemacht und mit Ether extrahiert; die Etherphase wird über Natriumsulfat getrocknet. Dann wird mit salzsaurem Ethanol das Salz gebildet. Man erhält so KHL-8430; F. 172-173 °C.

Beispiel 8

Zu 15,8 g der Base 2,6,6-Triphenyl-3-azahexan (Fendilin) werden unter Kühlen 5,9 g 98-100 %ige Ameisensäure gegeben; dann setzt man dem so entstandenen dicken Öl innerhalb von 5 min 5,7 g 30 %ige wässerige Formaldehydlösung zu. Das Gemisch wird bis zum Beginn der Gasentwicklung auf 40 °C gehalten, dann wird das Erhitzen 30 min lang unterbrochen. Danach wird das Gemisch 12 h lang bei 80 °C gerührt und anschließend eingedampft. Der Rückstand wird mit 100 ml 5 %iger Salzsäurelösung verrieben, 10 min lang im Wasserbad erwärmt und danach unter Kühlen alkalisch gemacht. Die Base wird mit Chloroform extrahiert, getrocknet, filtriert und eingedampft; der Rückstand wird in Ether gelöst und mit salzsaurem Ether in das Salz übergeführt. Man erhält so 14,2 g 3-Methyl-2,6,6-triphenyl-3-azahexan-hydrochlorid; F. 96 °C.

Beispiel 9

Ein Gemisch aus 30,0 g 1,5,5-Triphenyl-3-azapentan, 24,5 g 1-Brom-1-(3,4-dimethoxyphenyl)-ethan (Bull. Soc. Chim. France, 1973, 2665), 15,0 g Kaliumcarbonat und 100 ml Dimethylformamid wird 16 h lang bei 70 °C gerührt. Dann wird filtriert und das Lösungsmittel abdestilliert; der Rückstand wird in 100 ml Essigsäure mit 0,5 g Platinoxid bei 70 °C und 0,4 Pa hydriert, danach mit 100 ml Methanol verdünnt und filtriert, worauf das Lösungsmittel abdestilliert wird. Aus dem Rückstand wird gemäß Beispiel 1 (2a) das Salz gebildet. Man erhält 30,0 g KHL-8430; F. 171-173 °C (aus wässerigem Ethanol).

Beispiel 10

Zu einem Gemisch aus 21,0 g 1,1-Diphenyl-3-aminopropan und 10,5 g Natriumhydrogencarbonat werden innerhalb von 2 h 30 ml einer Lösung von 24,5 g 1-Brom-1-(3,4-dimethoxyphenyl)-ethanin Butanol unter Sieden und Rühren zugetropft. Dann wird bis zur Beendigung der Gasentwicklung weiter erhitzt; nach dem Abkühlen und Filtrieren wird das Lösungsmittel abdestilliert. Aus dem Rückstand wird in Etherlösung mit salzsaurem Ethanol das Salz gebildet, das dreimal mit wässerigem Ethanol kristalliert wird. Man erhält so 18,0 g KHL-8430; F. 171-173 °C.

Beispiele 11-53

Nach den in den Beispielen 1 (a1) und 1 (a2) beschriebenen Verfahren und mit der gleichen Ausbeute können die in der Tabelle 2 angeführten Verbindungen der allgemeinen Formel I (mit $R^1$ = H) hergestellt werden. In der Tabelle sind die Bedeutung von $R^2$ und Z, die salzbildende Säure, der Schmelzpunkt und die Literaturstellen zur Herstellung der Ketonkomponente, sofern diese nach einem in der Literatur erwähnten Verfahren hergestellt wurde, aufgeführt.

Tabelle 2

| Beispiel Nr. | $R^2$ | Z | Säure | F. (°C) | Herstellung der Ketonkomponente |
|---|---|---|---|---|---|
| 11 | H | 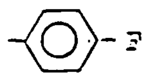 | HCl | 176-8 | J.A.C.S. 63 (1941) 974 |
| 12 | H | 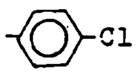 | HCl | 178 | J.Chem.Soc.1947,231 |
| 13 | H | 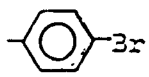 | HCl | 168 | Org.Synth.Coll.. Voll. I. 109 (1932) |
| 14 | H | 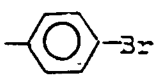 | Maleinsäure | 142 | Org.Synth.Coll. Vol. I 109 (1932) |
| 15 | H | 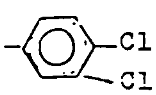 | HCl | 188 | J.Chem.Soc.1927, 1855 |
| 16 | H | 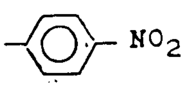 | HCl | 197-8 | J.A.C.S.68 (1946) 1386 |

| Beispiel Nr. | $R^2$ | Z | Säure | F. (°C) | Herstellung der Ketonkomponente |
|---|---|---|---|---|---|
| 17 | H | 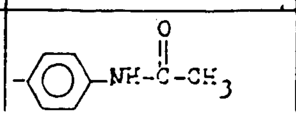 | HCl | 227-8 | Chem.Ber.42 (1909) 3482 |
| 18 | H | 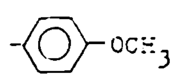 | HCl | 194-6 | J.Chem.Soc.1924, 202 |
| 19 | H | 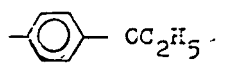 | HCl | 136-8 | J.A.C.S.76 (1954) 5150 |
| 20 | H | 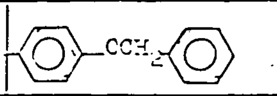 | HCl | 208-9 | J.Org.Chem.5 (1940) 355 |
| 21 | H | 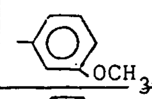 | HCl | 160-2 | J.Chem.Soc.1943, 499 |
| 22 | H | 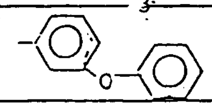 | HCl | 175 | J.A.C.S.58 (1936) 1810 |
| 23 | H | 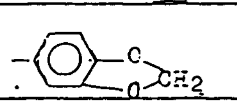 | HCl | 215-220 | Chem.Ber.36 (1903) 3595 |
| 24 | H | 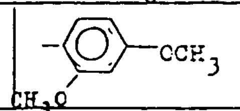 | HCl | 186-8 | Chem.Ber.24 (1891) 2461 |
| 25 | H | 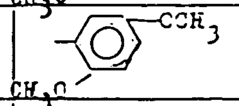 | HCl | 187-9 | Org.Synth.31 (1951) 90 |
| 26 | H | 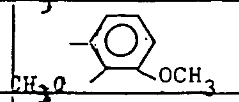 | HCl | 178-182 | J.A.C.S.52 (1930) 3718 |
| 27 | H | 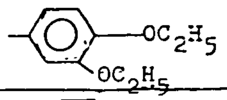 | HCl | 171-2 | Gazetta Chim. Ital. 77 (1947) 470 |
| 28 | H | 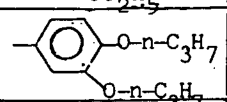 | HCl | 149-151 | |
| 29 | H | 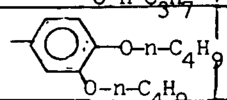 | HCl | 129-132 | J.Am.Pharm.Assoc.46 (1957) 544 |
| 30 | H | 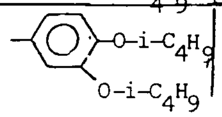 | HCl | 182-184 | |

| Beispiel Nr. | $R^2$ | Z | Säure | F. (°C) | Herstellung der Ketonkomponente |
|---|---|---|---|---|---|
| 31 | H | 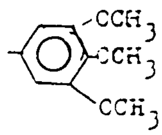 | HCl | 201-3 | J.A.C.S. 68 (1946) 1386 |
| 32 | H | 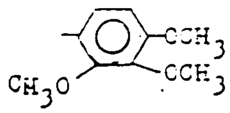 | HCl | 198-200 | Chem.Ber. 44 (1911) 1551 |
| 33 | H | 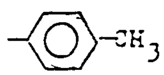 | HCl | 180-1 | J.A.C.S. 46 (1924) 1892 |
| 34 | H | 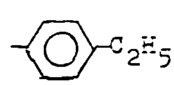 | HCl | 188-9 | J.A.C.S. 68 (1946) 1107 |
| 35 | H | 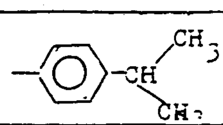 | HCl | 181-3 | Chem.Ber. 21 (1983) 2225 |
| 36 | H | 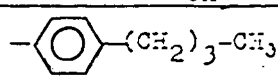 | HCl | 124-7 | Chem. Ber. 68 (1935) 1831 |
| 37 | H | 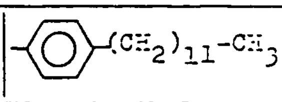 | HCl | 103-112 | J.Org.Chem. 20 (1953) 520 |
| 38 | H | 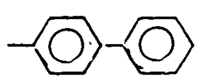 | HCl | 202-5 | J.A.C.S. 52 (1930) 3718 |
| 39 | H | 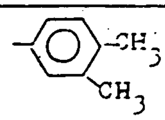 | HCl | 199-201 | J.A.C.S. 64 (1942) 423 |
| 40 | H | 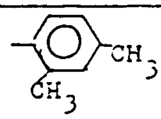 | HCl | 236-8 | J.A.C.S. 59 (1937) 804 |
| 41 | H | 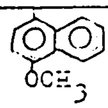 | HCl | 226-7 | Chem.Ber.47 (1914) 3222 |
| 42 | H | 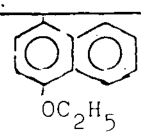 | HCl | 212-4 | Chem.Ber.47 (1914) 3222 |

| Beispiel Nr. | $R^2$ | Z | Säure | F. (°C) | Herstellung der Ketonkomponente |
|---|---|---|---|---|---|
| 43 | H | 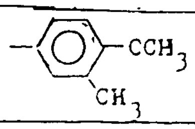 | HCl | 181-3 | J.A.C.S. 79 (1937) 3585 |
| 44 | H | 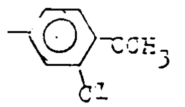 | HCl | 192-194 | J.Ind.Chem.Soc. 36 (1959) 786 |
| 45 | H | 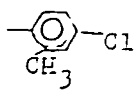 | HCl | 228-9 | Chem.Ber.65 (1932) 1297 |
| 46 | H | 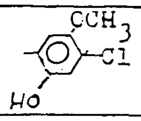 | HCl | 197-9 | Indian J. Chem.2(7), 296 |
| 47 | $CH_3$ | 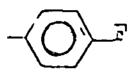 | Maleinsäure | 141-3 | J.Org.Chem.11 (1946) 444 |
| 48 | $CH_3$ | 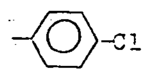 | HCl | 200 | J.Org.Chem.11 (1946) 444 |
| 49 | $CH_3$ | 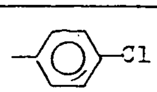 | Maleinsäure | 124-6 | J.Org.Chem.11 (1946) 444 |
| 50 | $CH_3$ | 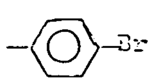 | HCl | 217-8 | J.Org.Chem.11 (1946) 444 |
| 51 | $CH_3$ | 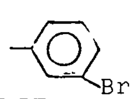 | HCl | 213 | J.Org.Chem 11 (1946) 444 |
| 52 | $CH_3$ | 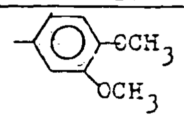 | HCl | 140-2 | J.A.C.S. 63 (1941) 531 |
| 53 | $n$-$C_{10}H_{21}$ | 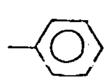 | HCl | 98-101 | J.Org.Chem.8 (1943) 139 |

Die in den Beispielen 28 und 30 verwendeten Ketonkomponenten wurden wie folgt hergestellt:

3,4-Diisobutoxyacetophenon

Zu einer mit 200 ml Dichlormethan hergestellten Suspension von 21,33 g (0,16 mol) wasserfreiem Aluminiumchlorid werden 33,35 g (0,15 mol) 1,2-Diisobutoxybenzol und dann 14,72 g (0,187 mol) Acetylchlorid zugetropft. Die so erhaltene Lösung wird bis zur Beendigung der Gasentwicklung im Wasserbad erhitzt und dann auf 20 ml konzentrierte Salzsäure enthaltendes Eis gegossen. Nach der Trennung werden die wässerige Phase mit Dichlormethan und die vereinigten organischen Phasen mit 2 N Natriumhydroxid und dann mit Wasser gewaschen. Die Dichlormethanlösung wird über Natriumsulfat getrocknet und

eingedampft; der Rückstand wird abdestilliert. Man erhält so 12,10 g (30,5 %) 3,4-Diisobutoxyacetophenon; Kp. 148 - 153 ° C bei einem Druck von 50 Pa (0,3 Torr). Das Produkt kann aus Hexan kristallisiert werden; F. 72 - 74 ° C.

3,4-Dipropoxyacetophenon

Zu einer mit 250 ml Dichlormethan bereiteten Suspension von 29,33 g (0,22 mol) wasserfreiem Aluminiumchlorid werden unter Eiskühlung 38,85 g (0,2 mol) 1,2-Dipropoxybenzol und dann 19,63 g (0,25 mol) Acetylchlorid zugetropft. Die Lösung wird danach 1,5 h lang erhitzt, dann abgekühlt und auf 25 ml konzentrierte Salzsäure enthaltendes Eis gegossen. Nach der Trennung werden die wässerige Phase mit Dichlormethan und die vereinigten organischen Phasen mit 2 N Natriumhydroxid und dann mit Wasser gewaschen. Nach dem Trocknen, Eindampfen und Destillieren erhält man 27,3 g (57 %) 3,4-Dipropoxyacetophenon; Kp. 150 - 156 ° C bei einem Druck von 90 Pa (0,5 Torr). Das Produkt kann aus Hexan kristallisiert werden; F. 51 - 53 ° C.

Beispiele 54 - 69

Nach dem in Beispiel 1 (a1) beschriebenen Verfahren können mit der gleichen Ausbeute die in der folgenden Tabelle 3 aufgeführten Verbindungen der allgemeinen Formel XIII mit $R^2$ = H hergestellt werden. In der Tabelle sind die Bedeutung von Z und der Schmelzpunkt angegeben.

Tabelle 3

| Beispiel Nr. | Z | F. (°C) |
|---|---|---|
| 54 | 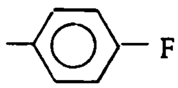 | 74 |
| 55 | 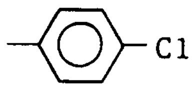 | 69 |
| 56 | 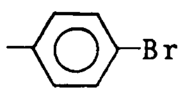 | 94 |
| 57 | 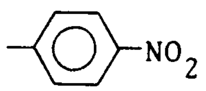 | 121 |
| 58 | 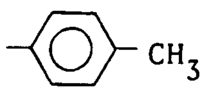 | 94 |
| 59 | 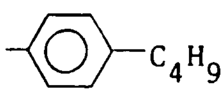 | 63 |
| 60 | 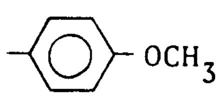 | 85 |
| 61 | 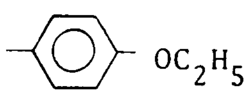 | 90 |

Tabelle 3 (Fortsetzung)

| Beisp. Nr. | Z | F. (°C) |
|---|---|---|
| 62 | 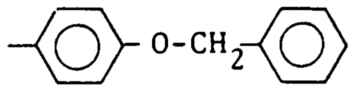 | 111 |
| 63 | 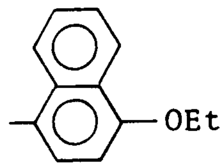 | 79 |
| 64 | 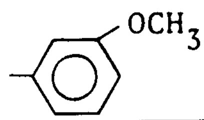 | 60 |
| 65 | 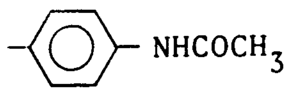 | 129 |
| 66 | 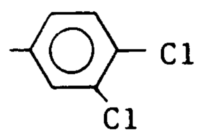 | 62 |
| 67 | 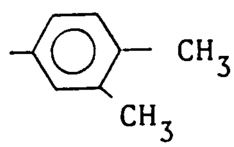 | 103 |
| 68 | 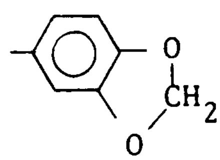 | 71 |
| 69 | 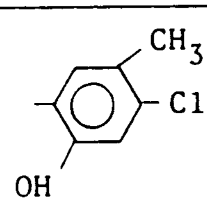 | 148 |

Beispiel 70

Auf die in Beispiel 8 beschriebene Weise wird aus 2-(3,4-Dimethoxyphenyl)-6,6-diphenyl-3-azahexan mit Ameisensäure und Formaldehyd mit einer Ausbeute von 86 % 2-(3,4-Dimethoxyphenyl)-3-methyl-6,6-diphenyl-3-azahexan-hydrochlorid hergestellt; F. 180 - 182 ° C.

Beispiel 71

5,68 g (0,02 mol) 3,3-Di-(4-fluorphenyl)-propylaminhydrochlorid (Span. 398, 516 C.A. 83 P 78 816 d) werden mit 20 ml 1 N Natriumhydroxid vermischt, dann mit Ether extrahiert und eingedampft. Die so gewonnene Base wird auf die in Beispiel 1 (a1) beschriebene Weise mit 3,6 g (0,02 mol) 3,4-Dimethoxyacetophenon umgesetzt. Aus der so erhaltenen Schiff'schen Base (F. 94 - 97 ° C) wird auf die in Beispiel 1 (a2) beschriebene Weise mit einer Ausbeute von 56 % das 2-(3,4-Dimethoxyphenyl)-6,6-di(4-fluorphenyl)-3-azahexan-hydrochlorid hergestellt; F. 174 - 176 ° C.

Beispiel 72

Arzneimittelzusammensetzungen

| a) Tabletten | |
|---|---|
| 2-(3,4-Dimethoxyphenyl)-6,6-diphenyl-3-azahexan-hydrochlorid | 100,0 g |
| Weizenstärke | 130,0 g |
| Calciumphosphat | 209,0 g |
| Magnesiumstearat | 1,0 g |
| | 440,0 g. |

Das pulverisierte Gemisch wird auf an sich bekannte Weise zu 1000 Stück Tabletten zu je 440 mg verpreßt. Jede Tablette enthält 100 mg Wirkstoff.

| b) Depotdragees | |
|---|---|
| 2-(3,4-Dimethoxyphenyl)-6,6-diphenyl-3-azahexan-hydrochlorid | 150,0 g |
| Carboxymethylcellulose | 300,0 g |
| Stearinsäure | 20,0 g |
| Celluloseacetat-phthalat | 30,0 g |
| | 500,0 g. |

Der Wirkstoff, die Carboxymethylcellulose und die Stearinsäure werden mit 200 ml einer Ethylacetat-Ethanol-Lösung von Celluloseacetat-phthalat gründlich verrieben, zu Dragees zu je 500 mg verpreßt und auf an sich bekannte Weise mit zuckerhaltigem 5 %-igen wässerigen Poly(vinylpyrrolidon) überzogen. Jedes Dragee enthält 150 mg Wirkstoff.

| c) Injektionslösungen | |
|---|---|
| 2-(3,4-Dimethoxyphenyl)-6,6-diphenyl-3-azahexan-hydrochlorid | 15,0 g |
| Destilliertes Wasser | ad 1000,0 $cm^3$. |

Der Wirkstoff wird auf an sich bekannte Weise in destilliertem Wasser gelöst; aus der Lösung werden 1000 Injektionslösungen zu jeweils 1 ml hergestellt, die jeweils 15 mg Wirkstoff enthalten.

**Patentansprüche**

1. Diphenylpropylamin-Derivate der allgemeinen Formel I,

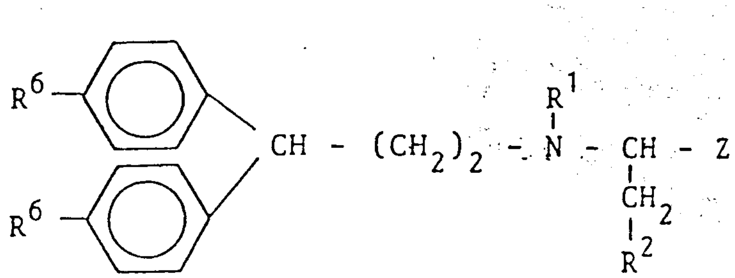

(I),

worin bedeuten:
$R^1$ Wasserstoff oder Methyl,
$R^2$ Wasserstoff, Methyl oder n-Decyl,
Z (a) eine durch $R^3$, $R^4$ und $R^5$ substituierte Phenylgruppe

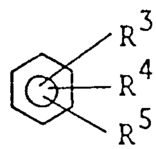

,

wobei bedeuten:
$R^3$ Wasserstoff, Fluor, Chlor oder Brom, Nitro, $C_{1-12}$-Alkyl, $C_{1-4}$-Alkoxy, Phenoxy oder Benzyloxy,
$R^4$ und $R^5$ Wasserstoff, Chlor, Hydroxy, Alkoxy, Benzyloxy, Acetamino oder Carboxy oder
$R^4$ und $R^5$ zusammen Methylendioxy oder
(b) 4-Methoxynaphthyl oder 4-Ethoxynaphthyl und
$R^6$ Wasserstoff oder Fluor,
und ihre Säureadditionssalze,
wobei solche 2-phenylsubstituierten Verbindungen der Formel I ausgenommen sind, bei denen
(A) $R^1$, $R^2$, $R^3$, $R^4$, $R^5$ und $R^6$ jeweils Wasserstoff
oder
(B) $R^1$, $R^3$, $R^4$, $R^5$ und $R^6$ jeweils Wasserstoff,
und
$R^2$ Methyl
bedeuten.

2. 2-(Diphenyl-4-yl)-6,6-diphenyl-3-azahexan der Formel

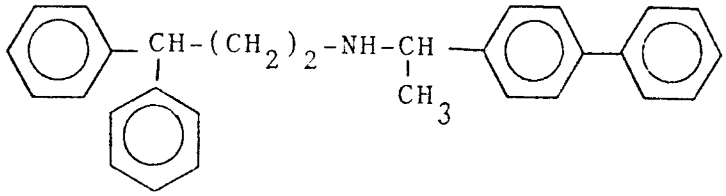

.

**3.** 2-(3,4-Dimethylphenyl)-6,6-diphenyl-3-azahexan der Formel

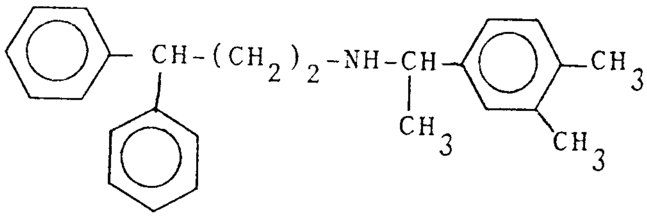

**4.** 2-(2,4-Dimethylphenyl)-6,6-diphenyl-3-azahexan der Formel

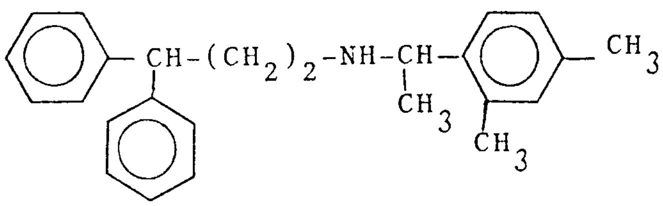

**5.** Verbindungen gemäß Anspruch 1:
2-(3,4-Dimethoxyphenyl)-6,6-diphenyl-3-azahexan,
2-(4-Fluorphenyl)-6,6-diphenyl-3-azahexan,
2-(4-Chlorphenyl)-6,6-diphenyl-3-azahexan,
2-(4-Bromphenyl)-6,6-diphenyl-3-azahexan,
2-(3,4-Dichlorphenyl)-6,6-diphenyl-3-azahexan,
2-(4-Acetaminophenyl)-6,6-diphenyl-3-azahexan,
2-(4-Methoxyphenyl)-6,6-diphenyl-3-azahexan,
2-(4-Ethoxyphenyl)-6,6-diphenyl-3-azahexan,
2-(4-Benzyloxyphenyl)-6,6-diphenyl-3-azahexan,
2-(3-Methoxyphenyl)-6,6-diphenyl-3-azahexan,
2-(3-Phenoxyphenyl)-6,6-diphenyl-3-azahexan,
2-(3,4-Methylendioxyphenyl)-6,6-diphenyl-3-azahexan,
2-(2,4-Dimethoxyphenyl)-6,6-diphenyl-3-azahexan,
2-(2,5-Dimethoxyphenyl)-6,6-diphenyl-3-azahexan,
2-(2,3-Dimethoxyphenyl)-6,6-diphenyl-3-azahexan,
2-(3,4-Diethoxyphenyl)-6,6-diphenyl-3-azahexan,
2-[3,4-Di(propan-2-yloxy)-phenyl]-6,6-diphenyl-3-azahexan,
2-[3,4-Di-(propan-1-yloxy)-phenyl]-6,6-diphenyl-3-azahexan,
2-[3,4-Di-(2-methylpropan-1-yloxy)-phenyl]-6,6-diphenyl-3-azahexan,
2-[3,4-Di-(butan-1-yloxy)-phenyl]-6,6-diphenyl-3-azahexan,
2-(3,4,5-Trimethoxyphenyl)-6,6-diphenyl-3-azahexan,
2-(2,3,4-Trimethoxyphenyl)-6,6-diphenyl-3-azahexan,
2,(4- Methylphenyl)-6,6-diphenyl-3-azahexan,
2-(4-Ethylphenyl)-6,6-diphenyl-3-azahexan,
2-[4-(Propan-2-yl)-phenyl]-6,6-diphenyl-3-azahexan,
2-[4-(Dodecan-1-yl)-phenyl]-6,6-diphenyl-3-azahexan,
2-(4-Methoxynaphthyl-1)-6,6-diphenyl-3-azahexan,
2-(4-Ethoxynaphthyl-1)-6,6-diphenyl-3-azahexan,
2-(3-Methyl-4-methoxyphenyl)-6,6-diphenyl-3-azahexan,
2-(3-Chlor-4-methoxyphenyl)-6,6-diphenyl-3-azahexan,
2-(4-Chlor-2-methylphenyl)-6,6-dipheny-3-azahexan,

2-(4-Chlor-2-hydroxy-5-methoxyphenyl)-6,6-diphenyl-3-azahexan,
3-(4-Fluorphenyl)-7,7-diphenyl-4-azaheptan,
3-(4-Chlorphenyl)-7,7-diphenyl-4-azaheptan,
3-(4-Bromphenyl)-7,7-diphenyl-4-azaheptan,
3-(3-Bromphenyl)-7,7-diphenyl-4-azaheptan,
3-(3,4-Dimethoxyphenyl)-7,7-diphenyl-4-azaheptan,
1,1,5-Triphenyl-4-azahexadecan,
2-(3,4-Dimethoxyphenyl)-3-methyl-6,6-diphenyl-3-azahexan,
3-Methyl-2,6,6-triphenyl-3-azahexan,
2-(3,4-Dimethoxyphenyl)-6,6-di-(4-fluorphenyl)-3-azahexan,
2-[4-(Butan-1-yl)-phenyl]-6,6-diphenyl-3-azahexan.

**6.** Verfahren zur Herstellung der Diphenylpropylamin-Derivate der allgemeinen Formel I bzw. ihrer Säureadditionssalze nach Anspruch 1,
**gekennzeichnet** durch folgende Maßnahmen:

(A) zur Herstellung von Verbindungen der allgemeinen Formel I mit $R^1$ = H:

(A1) an sich bekannte einstufige oder zweistufige reduktive Kondensation eines Diphenylpropylamins der Formel II,

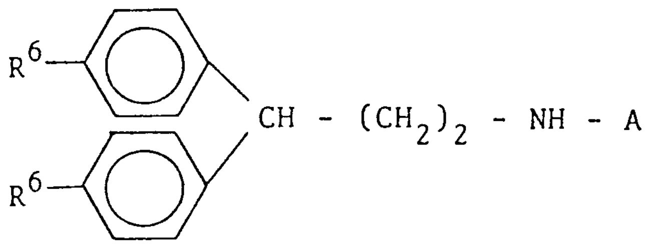

(II),

worin A Wasserstoff und $R^6$ Wasserstoff oder Fluor bedeuten,
mit einem Keton der allgemeinen Formel III

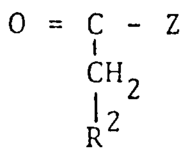

(III)

mit $R^2$ und Z wie in Anspruch 1;
oder
(A2) Umsetzung einer Verbindung der allgemeinen Formel IV,

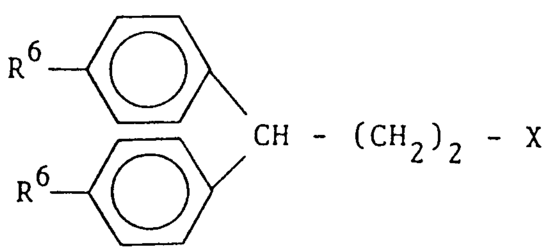

(IV),

worin X Halogen, vorzugsweise Chlor, Brom oder Jod, und $R^6$ Wasserstoff oder Fluor bedeuten,
mit einem Amin der allgemeinen Formel V

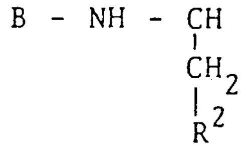

(V),

worin B Wasserstoff oder Benzyl und $R^2$ und Z dasselbe wie in Anspruch 1 bedeuten,
und
Debenzylierung der erhaltenen Verbindung, wenn B Benzyl ist;
oder
(A3) Umsetzung eines Amins der allgemeinen Formel II, worin A Wasserstoff oder Benzyl und $R^6$ Wasserstoff oder Fluor bedeuten, mit einer Verbindung der allgemeinen Formel VI,

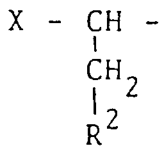

(VI),

worin $R^2$, Z und X die beim Verfahren A2 angegebene Bedeutung besitzen,
und
Debenzylierung der erhaltenen Verbindung, wenn A Benzyl ist;
oder
(A4) an sich bekannte einstufige oder zweistufige reduktive Kondensation eines Diphenylpropionaldehyds der Formel VII,

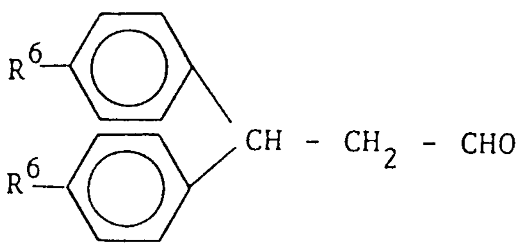

(VII),

worin $R^6$ Wasserstoff oder Fluor bedeutet,
mit einem Amin der Formel V, worin B Wasserstoff und $R^2$ und Z dasselbe wie in Anspruch 1 bedeuten;
oder
(A5) an sich bekannte Umsetzung eines Diphenylacetonitrils der Formel VIII,

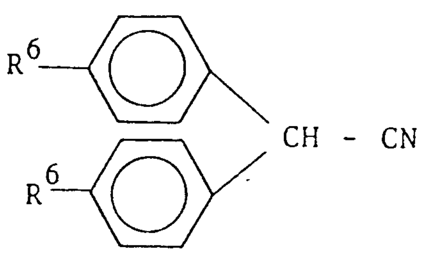

(VIII),

worin $R^6$ Wasserstoff oder Fluor bedeutet,
mit einer Verbindung der allgemeinen Formel IX,

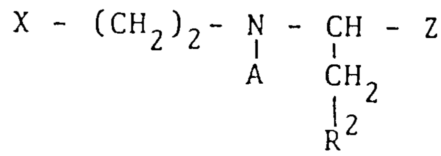

(IX),

worin $R^2$, Z und X die bei Verfahren A2 angegebene Bedeutung besitzen und A Benzyl ist,
und
Austausch der Cyanogruppe und der Benzylgruppe gegen Wasserstoff bei der so erhaltenen Verbindung;
oder
(A6) Umsetzung eines Amins der allgemeinen Formel X,

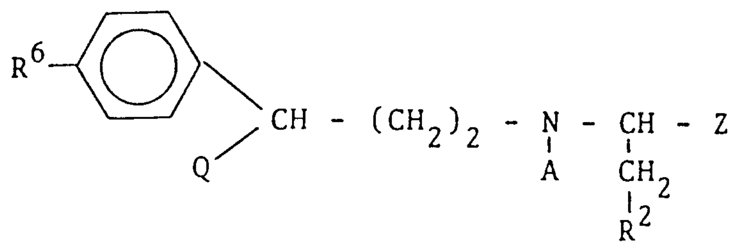

(X),

worin Q Hydroxy oder Halogen, A Wasserstoff oder Benzyl, $R^2$, $R^6$ und Z dasselbe wie in Anspruch 1 bedeuten,
oder eines Salzes davon in einer Friedel-Crafts-Reaktion mit Benzol oder Fluorbenzol
und
Debenzylierung der erhaltenen Verbindung, wenn A Benzyl ist;
oder

(B) zur Herstellung von Verbindungen der allgemeinen Formel I mit $R^1$ = Methyl:
(B1) N-Methylierung einer Verbindung der allgemeinen Formel I mit $R^1$ = H in an sich bekannter Weise;
oder
(B2) Umsetzung eines Amins der Formel XI,

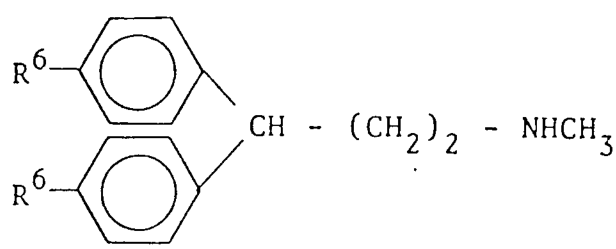

(XI),

worin $R^6$ Wasserstoff oder Fluor bedeutet,
mit einer Verbindung der allgemeinen Formel VI, worin $R^2$, X und Z die beim Verfahren A2 angegebne Bedeutung besitzen;
oder

(B3) Umsetzung eines Amins der allgemeinen Formel XII,

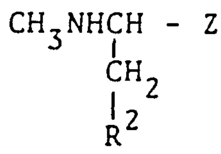

(XII),

worin Z und $R^2$ dasselbe wie in Anspruch 1 bedeuten, mit einer Verbindung der allgemeinen Formel IV, worin X die beim Verfahren A2 angegebene Bedeutung besitzt, und gewünschtenfalls Oberführung der jeweils erhaltenen Verbindung der Formel I mit anorganischen oder organischen Säuren in ein entsprechendes Säureadditionssalz.

**7.** Verfahren zur Herstellung der Diphenylpropylamin-Derivate nach den Ansprüchen 2 bis 4, **gekennzeichnet** durch
au sich bekannte zweistufige reduktive Kondensation von 3,3-Diphenylpropylamin der Formel IIa

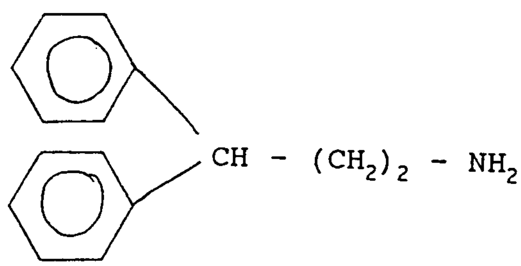

(IIa)

mit einem Keton der allgemeinen Formel IIIa

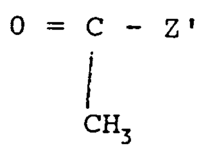

(IIIa)

in der Z' Diphenyl-4-yl, 3,4-Dimethylphenyl oder 2,4-Dimethylphenyl bedeutet, und gewünschtenfalls Überführung der jeweils erhaltenen Verbindung mit anorganischen oder organischen Säuren in ein entsprechendes Säureadditionssalz.

**8.** Pharmazeutische Mittel,
**gekennzeichnet durch**
eine oder mehrere Verbindungen nach den Ansprüchen 1 bis 5 und/oder physiologisch verträgliche Säureadditionssalze davon als Wirkstoffe zusammen mit inerten, üblichen Hilfs- und/oder Trägerstoffen.

**9.** Verwendung der Verbindungen nach den Ansprüchen 1 bis 5 zur Herstellung pharmazeutischer Mittel zur Behandlung von Herz- und Kreislauferkrankungen.

## Claims

**1.** Diphenylpropylamine derivatives of general formula I

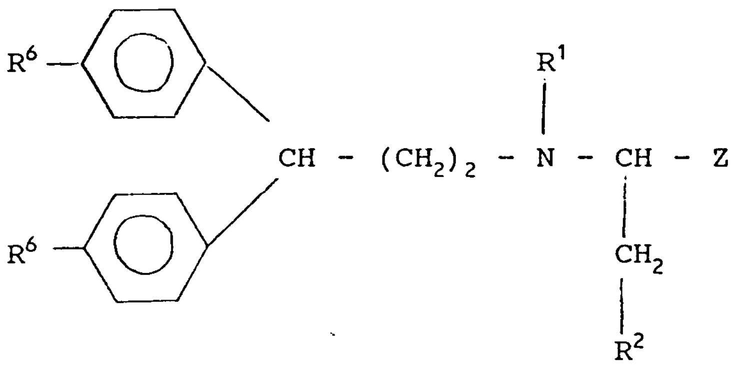

(I)

wherein:
$R^1$ represents hydrogen or methyl,
$R^2$ represents hydrogen, methyl or n-decyl,
Z represents (a) a phenyl group substituted by $R^3$, $R^4$ and $R^5$

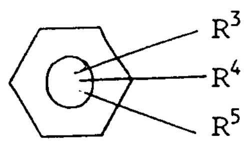

wherein:
$R^3$ represents hydrogen, fluorine, chlorine or bromine, nitro, $C_{1-12}$-alkyl, $C_{1-4}$-alkoxy, phenoxy or benzyloxy,
$R^4$ and $R^5$ represent hydrogen, chlorine, hydroxy, alkoxy, benzyloxy, acetamino or carboxy or
$R^4$ and $R^5$ together represent methylenedioxy or
(b) 4-methoxynaphthyl or 4-ethoxynaphthyl and
$R^6$ represents hydrogen or fluorine,
and the acid addition salts thereof,
with the exception of those 2-phenyl-substituted compounds of formula I wherein
(A) $R^1$, $R^2$, $R^3$, $R^4$, $R^5$ and $R^6$ each represent hydrogen or
(B) $R^1$, $R^3$, $R^4$, $R^5$ and $R^6$ each represent hydrogen and $R^2$ represents methyl.

**2.** 2-(Diphenyl-4-yl)-6,6-diphenyl-3-azahexane of the formula

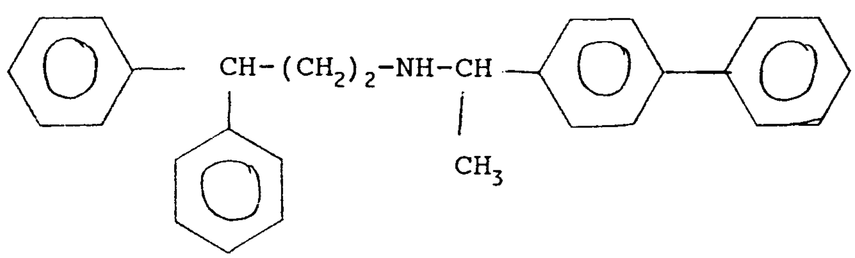

**3.** 2-(3,4-Dimethylphenyl)-6,6-diphenyl-3-azahexane of the formula

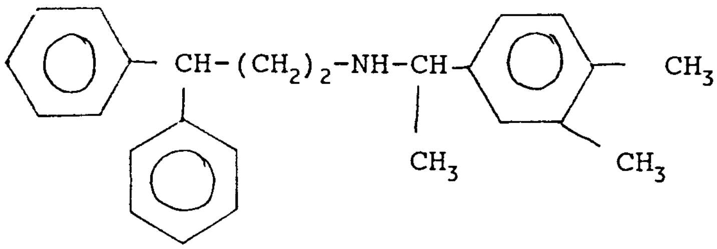

**4.** 2-(2,4-Dimethylphenyl)-6,6-diphenyl-3-azahexane of the formula

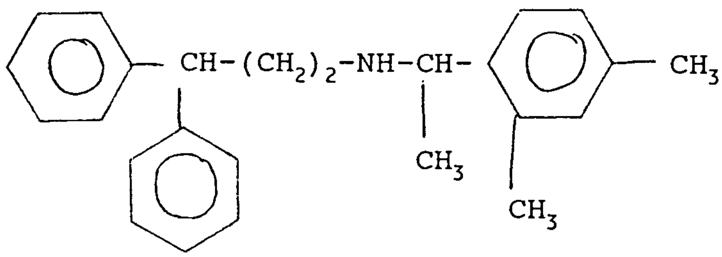

**5.** Compounds according to claim 1:
2-(3,4-dimethoxyphenyl)-6,6-diphenyl-3-azahexane,
2-(4-fluorophenyl)-6,6-diphenyl-3-azahexane,
2-(4-chlorophenyl)-6,6-diphenyl-3-azahexane,
2-(4-bromophenyl)-6,6-diphenyl-3-azahexane,
2-(3,4-dichlorophenyl-6,6-diphenyl-3-azahexane,
2-(4-acetaminophenyl)-6,6-diphenyl-3-azahexane,
2-(4-methoxyphenyl)-6,6-diphenyl-3-azahexane,
2-(4-ethoxyphenyl)-6,6-diphenyl-3-azahexane,
2-(4-benzyloxyphenyl)-6,6-diphenyl-3-azahexane,
2-(4-methoxyphenyl)-6,6-diphenyl-3-azahexane,
2-(4-phenoxyphenyl)-6,6-diphenyl-3-azahexane,
2-(3,4-methylenedioxyphenyl)-6,6-diphenyl-3-azahexane,
2-(2,4-dimethoxyphenyl)-6,6-diphenyl-3-azahexane,
2-(2,5-dimethoxyphenyl)-6,6-diphenyl-3-azahexane,
2-(2,3-dimethoxyphenyl)-6,6-diphenyl-3-azahexane,
2-(3,4-diethoxyphenyl)-6,6-diphenyl-3-azahexane,
2-[3,4-di(propan-2-yloxy)-phenyl]-6,6-diphenyl-3-azahexane,
2-[3,4-di-(propan-1-yloxy)-phenyl]-6,6-diphenyl-3-azahexane,
2-[3,4-di-(2-methylpropan-1-yloxy)-phenyl]-6,6-diphenyl-3-azahexane,
2-[3,4-di-(butan-1-yloxy)-phenyl]-6,6-diphenyl-3-azahexane,
2-(3,4,5-(trimethoxyphenyl]-6,6-diphenyl-3-azahexane,
2-(2,3,4-(trimethoxyphenyl]-6,6-diphenyl-3-azahexane,
2,(4-(methylphenyl)-6,6-diphenyl-3-azahexane,
2-(4-(ethylphenyl)-6,6-diphenyl-3-azahexane,
2-[4-(propan-2-yl)phenyl]-6,6-diphenyl-3-azahexane,
2-[4-(dodecan-1-yl)phenyl]-6,6-diphenyl-3-azahexane,
2-(4-methoxynaphthyl-l)-6,6-diphenyl-3-azahexane,
2-(4-ethoxynaphthyl-l)-6,6-diphenyl-3-azahexane,
2-(3-methyl-4-methoxyphenyl)-6,6-diphenyl-3-azahexane,
2-(3-chloro-4-methoxyphenyl)-6,6-diphenyl-3-azahexane,
2-(4-chloro-2-methylphenyl)-6,6-diphenyl-3-azahexane,

2-(4-chloro-2-hydroxy-5-methoxyphenyl)-6,6-diphenyl-3-azahexane,
3-(4-fluorophenyl)-7,7-diphenyl-4-azaheptane,
3-(4-chlorophenyl)-7,7-diphenyl-4-azaheptane,
3-(4-bromophenyl)-7,7-diphenyl-4-azaheptane,
3-(3-bromophenyl)-7,7-diphenyl-4-azaheptane,
3-(3,4-dimethoxyphenyl)-7,7-diphenyl-4-azaheptane,
1,1,5-triphenyl-4-azahexadecane,
2-(3,4-dimethoxyphenyl)-3-methyl-6,6-diphenyl-3-azahexane,
3-methyl-2,6,6-triphenyl-3-azahexane,
2-(3,4-dimethoxyphenyl)-6,6-di-(4-fluorophenyl-3-azahexane,
2-[4-(butan-1-yl)phenyl]-6,6-diphenyl-3-azahexane.

6. Process for preparing the diphenylpropylamine derivatives of general formula I or the acid addition salts thereof according to claim 1, characterised by the following procedures:

(A) in order to prepare compounds of general formula I wherein $R^1$ = H:

(A1) two-step reductive condensation, known per se, of a diphenylpropylamine of formula II

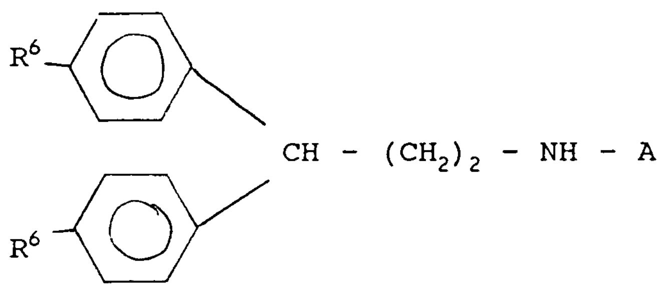

(II)

wherein A represents hydrogen and $R^6$ represents hydrogen or fluorine, with a ketone of general formula III

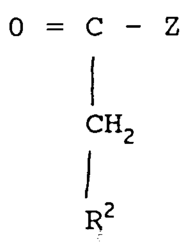

(III)

with $R^2$ and Z as defined in claim 1; or

(A2) reaction of a compound of general formula IV,

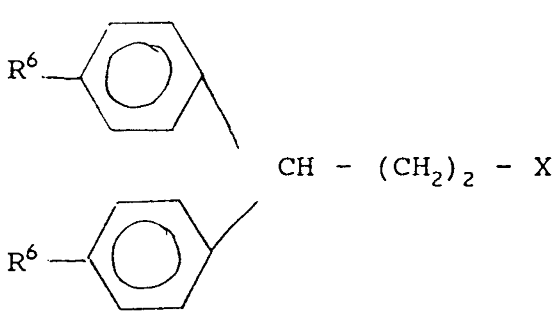

(IV)

wherein X represents halogen, preferably chlorine, bromine or iodine, and $R^6$ represents hydrogen or fluorine, with an amine of general formula V

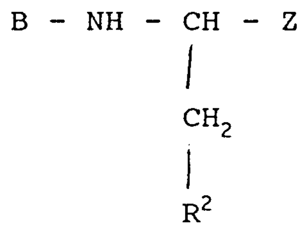

(V)

wherein B represents hydrogen or benzyl and $R^2$ and Z have the same meanings as in claim 1, and
debenzylation of the resulting compound if B represents benzyl; or
(A3) reaction of an amine of general formula II wherein A represents hydrogen or benzyl and $R^6$ represents hydrogen or fluorine, with a compound of general formula VI

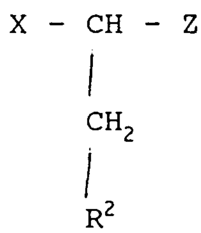

(VI)

wherein $R^2$, Z and X have the meanings given under process A2,
and
debenzylation of the resulting compound if A represents benzyl; or
(A4) one-step or two-step reductive condensation, known per se, of a diphenylpropionaldehyde of formula VII

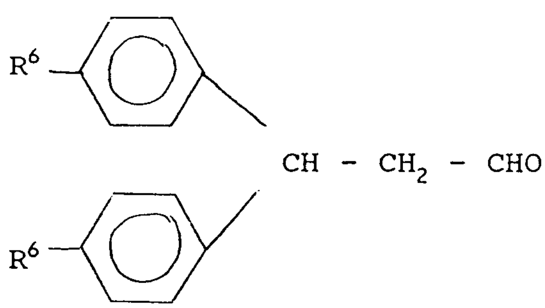

(VII)

wherein $R^6$ represents hydrogen or fluorine,
with an amine of formula V wherein B represents hydrogen and $R^2$ and Z have the same meanings as in claim 1; or

(A5) reaction, known per se, of a diphenylacetonitrile of formula VIII

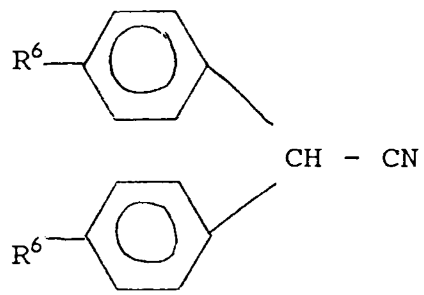

(VIII)

wherein $R^6$ represents hydrogen or fluorine, with a compound of general formula IX

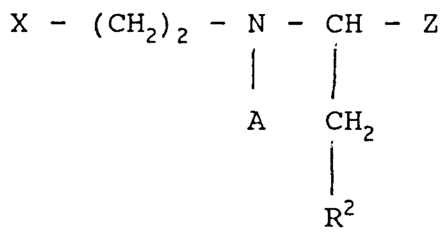

(IX)

wherein $R^2$, Z and X have the meanings given for process A2 and A represents benzyl, and exchanging the cyano group and benzyl group for hydrogen in the resulting compound; or
(A6) reaction of an amine of general formula X

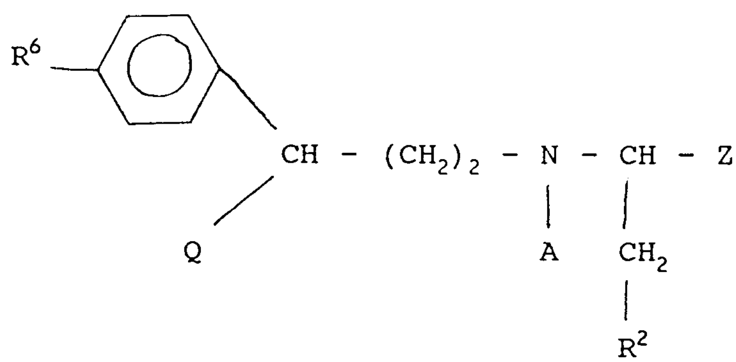

(X)

wherein Q represents hydroxy or halogen, A represents hydrogen or benzyl, $R^2$, $R^6$ and Z have the same meanings as in claim 1,
or a salt thereof in a Friedel-Crafts reaction with benzene or fluorobenzene and debenzylation of the resulting compound if A is benzyl;
or

(B) in order to prepare compounds of general formula I wherein $R^1$ = methyl:
(B1) N-methylation of a compound of general formula I wherein $R^1$ = H in a manner known per se; or

(B2) reaction of an amine of formula XI

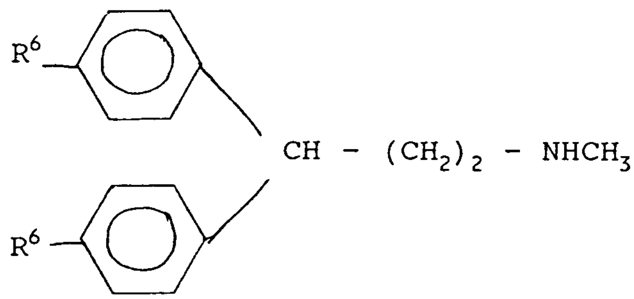

(XI)

wherein $R^6$ represents hydrogen or fluorine,
with a compound of general formula VI wherein $R^2$, X and Z have the meanings given in process A2; or
(B3) reaction of an amine of general formula XII

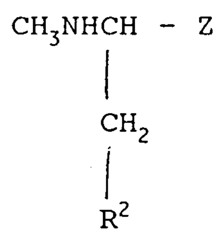

(XII)

wherein Z and $R^2$ have the same meanings as in claim 1, with a compound of general formula IV wherein X has the meaning given in process A2, and, if desired, conversion of the resulting compound of formula I with organic or inorganic acids into a corresponding acid addition salt.

7. Process for preparing the diphenylpropylamine derivatives according to claims 2 to 4, characterised by two-step reductive condensation, known per se, of 3,3-diphenylpropylamine of formula IIa

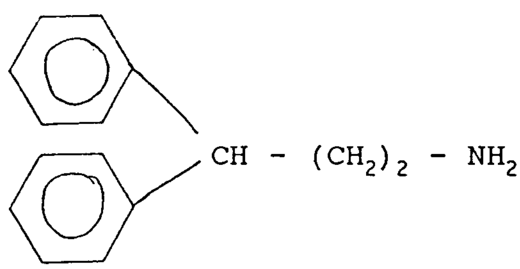

(IIa)

with a ketone of general formula IIIa

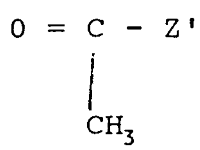

(IIIa)

wherein Z' represents diphenyl-4-yl, 3,4-dimethylphenyl or 2,4-dimethylphenyl,
and if desired conversion of the resulting compound with organic or inorganic acids into a corresponding acid addition salt.

8. Pharmaceutical compositions characterized by one or more compounds according to claims 1 to 5 and/or physiologically acceptable acid addition salts thereof as active substances together with inert conventional excipients and/or carriers.

9. Use of the compounds according to claims 1 to 5 for preparing pharmaceutical compositions for the treatment of cardiac and circulatory disorders.

**Revendications**

1. Dérivés de la diphénylpropylamine de formule générale I

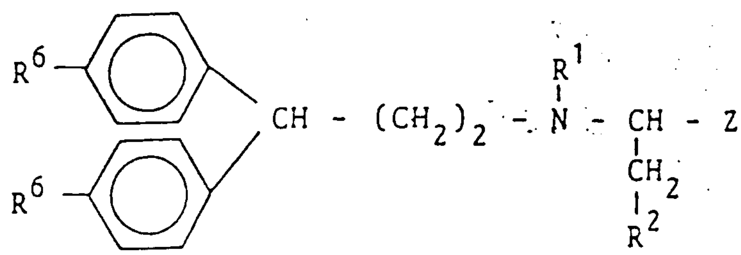

(I),

où
$R^1$ représente l'hydrogène ou le méthyle,
$R^2$ représente l'hydrogène, le méthyle ou le n-décyle,
Z représente
(a) un groupe phényle substitué par $R^3$, $R^4$ et $R^5$

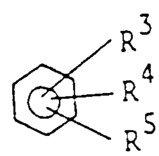

,

$R^3$ représentant l'hydrogène, le fluor, le chlore ou le brome, un groupe nitro, un alkyle en $C_{1-12}$, un alcoxy en $C_{1-4}$, le phénoxy ou le benzyloxy,
$R^4$ et $R^5$ représentant l'hydrogène, le chlore, l'hydroxy, un alcoxy, le benzyloxy, l'acétamino ou le carboxy ou
$R^4$ et $R^5$ représentant ensemble le méthylènedioxy ou
(b) le 4-méthoxynaphtyle ou le 4-éthoxynaphtyle et $R^6$ représente l'hydrogène ou le fluor, et leurs sels d'addition avec les acides,
à l'exclusion des composés de formule I 2-phényle substitués dans lesquels
(A) $R^1$, $R^2$, $R^3$, $R^4$, $R^5$ et $R^6$ représentent l'hydrogène,
ou
(B) $R^1$, $R^3$, $R^4$, $R^5$ et $R^6$ représentent l'hydrogène et $R^2$ le méthyle.

2. 2-(diphényl-4-yl)-6,6-diphényl-3-azahexane de formule

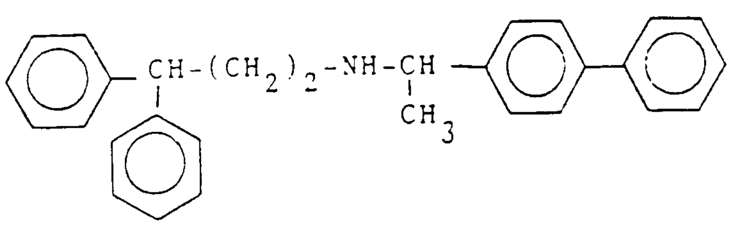

.

**3.** 2-(3,4-diméthylphényl)-6,6-diphényl-3-azahexane de formule

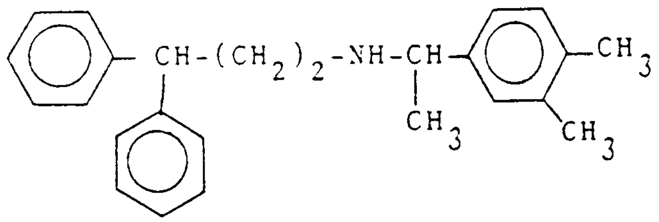

**4.** 2-(2,4-diméthylphényl)-6,6-diphényl-3-azahexane de formule

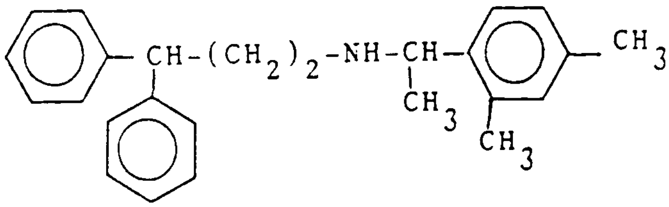

**5.** Composés selon la revendication 1 :
le 2-(3,4-diméthoxyphényl)-6,6-diphényl-3-azahexane,
le 2-(4-fluorophényl)-6,6-diphényl-3-azahexane,
le 2-(4-chlorophényl)-6,6-diphényl-3-azahexane,
le 2-(4-bromophényl)-6,6-diphényl-3-azahexane,
le 2-(3,4-dichlorophényl)-6,6-diphényl-3-azahexane,
le 2-(4-acétaminophényl)-6,6-diphényl-3-azahexane,
le 2-(4-méthoxyphényl)-6,6-diphényl-3-azahexane,
le 2-(4-éthoxyphényl)-6,6-diphényl-3-azahexane,
le 2-(4-benzyloxyphényl)-6,6-diphényl-3-azahexane,
le 2-(3-méthoxyphényl)-6,6-diphényl-3-azahexane,
le 2-(3-phénoxyphényl)-6,6-diphényl-3-azahexane,
le 2-(3,4-méthylènedioxyphényl)-6,6-diphényl-3-azahexane,
le 2-(2,4-diméthoxyphényl)-6,6-diphényl-3-azahexane,
le 2-(2,5-diméthoxyphényl)-6,6-diphényl-3-azahexane,
le 2-(2,3-diméthoxyphényl-6,6-diphényl-3-azahexane,
le 2-(3,4-diéthoxyphényl)-6,6-diphényl-3-azahexane,
le 2-[3,4-di(propan-2-yloxy)-phényl]-6,6-diphényl-3-azahexane,
le 2-[3,4-di(propan-1-yloxy)-phényl]-6,6-diphényl-3-azahexane,
le 2-[3,4-di-(2-méthylpropan-1-yloxy)-phényl]-6,6-diphényl-3-azahexane,
le 2-[3,4-di-(butan-1-yloxy)-phényl]-6,6-diphényl-3-azahexane,
le 2-(3,4,5-triméthoxyphényl)-6,6-diphényl-3-azahexane,
le 2-(2,3,4-triméthoxyphényl)-6,6-diphényl-3-azahexane,
le 2-(4-méthylphényl)-6,6-diphényl-3-azahexane,
le 2-(4-éthylphényl)-6,6-diphényl-3-azahexane,
le 2-[4-(propan-2-yl)-phényl]-6,6-diphényl-3-azahexane,
la 2-[4-(dodécan-1-yl)-phényl]-6,6-diphényl-3-azahexane,
le 2-(4-méthoxynaphtyl-1)-6,6-diphényl-3-azahexane,
le 2-(4-éthoxynaphtyl-1)-6,6-diphényl-3-azahexane,
le 2-(3-méthyl-4-méthoxyphényl)-6,6-diphényl-3-azahexane,
le 2-(3-chloro-4-méthoxyphényl)-6,6-diphényl-3-azahexane,
le 2-(4-chloro-2-méthylphényl)-6,6-diphényl-3-azahexane,
le 2-(4-chloro-2-hydroxy-5-méthoxyphényl)-6,6-diphényl-3-azahexane,

le 3-(4-fluorophényl)-7,7-diphényl-4-azaheptane,
le 3-(4-chlorophényl)-7,7-diphényl-4-azaheptane,
le 3-(4-bromophényl)-7,7-diphényl-4-azaheptane,
le 3-(3-bromophényl)-7,7-diphényl-4-azaheptane,
le 3-(3,4-diméthoxyphényl)-7,7-diphényl-4-azaheptane,
le 1,1,5-triphényl-4-azahexadécane,
le 2-(3,4-diméthoxyphényl)-3-méthyl-6,6-diphényl-3-azahexane,
le 3-méthyl-2,6,6-triphényl-3-azahexane,
le 2-(3,4-diméthoxyphényl)-6,6-di-(4-fluorophényl)-3-azahexane,
le 2[4(butan-1-yl)-phényl]-6,6-diphényl-3-azahexane.

**6.** Procédé pour la préparation des dérivés de la diphénylpropylamine de formule générale I ou de leurs sels d'addition avec les acides, selon la revendication 1, caractérisé par les dispositions suivantes :
(A) pour la préparation des composés de formule générale I avec R' = H.
($A_1$) condensation par voie réductrice, en une ou deux étapes, connue en soi, d'une diphénylpropylamine de formule II

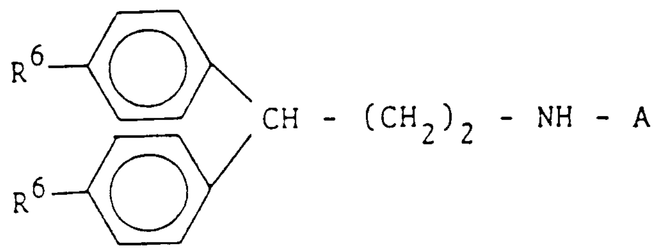

(II),

où A représente l'hydrogène et $R^6$ l'hydrogène ou le fluor, avec une cétone de formule générale III

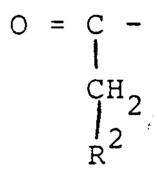

(III)

avec $R^2$ et Z tels que dans la revendication 1;
ou
($A_2$) réaction d'un composé de formule générale IV

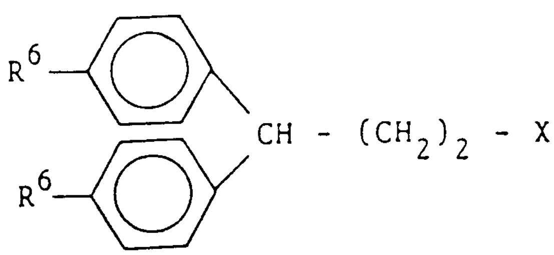

(IV),

où X représente un halogène, de préférence, le chlore, le brome ou l'iode, et $R^6$ représente l'hydrogène ou le fluor, avec une amine de formule générale V

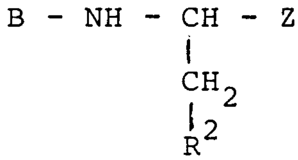 (V),

où B représente l'hydrogène ou le benzyle et $R^2$ et Z ont les mêmes significations que celles indiquées dans la revendication 1,
et
débenzylation du composé obtenu, lorsque B est le benzyle;
ou
(A3) réaction d'une amine de formule générale II où A représente l'hydrogène ou le benzyle et $R^6$ l'hydrogène ou le fluor, avec un composé de formule générale VI

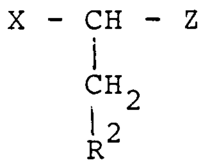 (VI),

où $R^2$, Z et X ont les significations indiquées pour le procédé A2,
et
débenzylation du composé obtenu, lorsque A est le benzyle;
ou
(A4) condensation par voie réductrice, en une ou deux étapes, connue en soi, d'un aldéhyde diphénylpropionique de formule VII

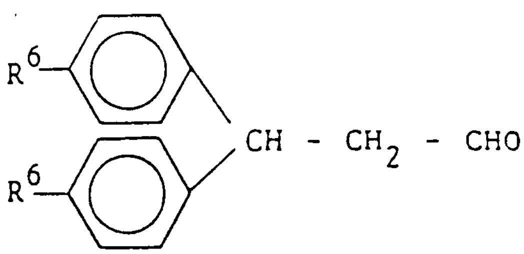 (VII),

où $R^6$ représente l'hydrogène ou le fluor,
avec une amine de formule V dans laquelle B représente l'hydrogène et $R^2$ et Z ont les mêmes significations que celles données dans la revendication 1;
ou
(A5) réaction connue en soi d'un diphénylacétonitrile de formule VIII

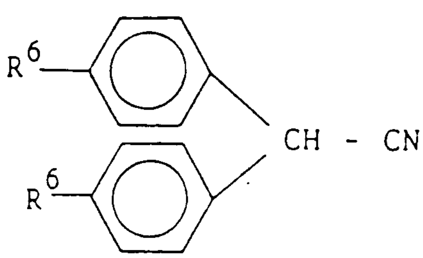 (VIII),

où $R^6$ représente l'hydrogène ou le fluor, avec un composé de formule générale IX

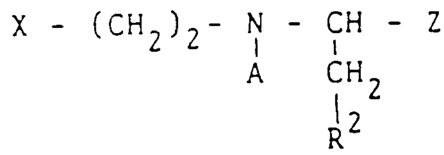 (IX),

où $R^2$, Z et X ont les significations indiquées pour le procédé A2 et A est le benzyle,
et
échange du groupe cyano et du groupe benzyle contre l'hydrogène dans le composé ainsi obtenu;
ou
(A6) réaction d'une amine de formule générale X

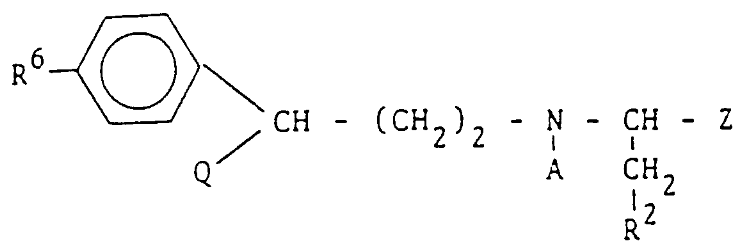 (X),

où Q représente l'hydroxy ou un halogène, A représente l'hydrogène ou le benzyle,
$R^2$, $R^6$ et Z ont les mêmes significations que celles données dans la revendication 1,
ou de l'un de ses sels dans une réaction de Friedel-Crafts avec le benzène ou le fluorobenzène et débenzylation du composé obtenu, lorsque A est le benzyle;
ou
(B) pour la préparation des composés de formule générale I avec $R^1$ = méthyle;
(B1) N-méthylation d'un composé de formule générale I avec $R^1$ = H, d'une façon connue en soi,
ou
(B2) réaction d'une amine de formule XI

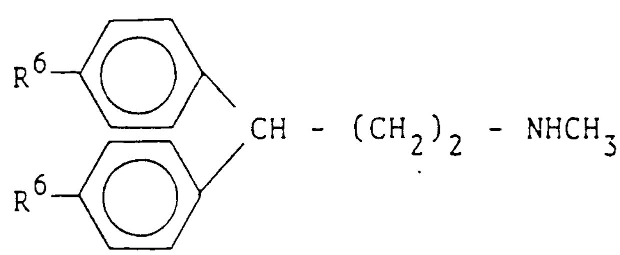 (XI),

où $R^6$ représente l'hydrogène ou le fluor,
avec un composé de formule générale VI dans laquelle $R^2$,
X et Z ont les significations indiquées pour le procédé A2;
ou

(B3) réaction d'une amine de formule générale XII

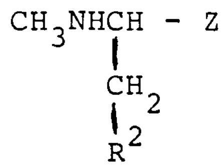

(XII),

où Z et $R^2$ ont les mêmes significations que celles données dans la revendication 1, avec un composé de formule générale IV dans laquelle X a la signification indiquée pour le procédé $A_2$, et, si désiré, transformation du composé de formule I obtenu avec des acides minéraux ou organiques en un sel d'addition d'acide correspondant.

7. Procédé pour la préparation des dérivés de la diphénylpropylamine selon les revendications 2 à 4, caractérisé par une condensation par voie réductrice, en une ou deux étapes, connue en soi, de 3,3-diphénylpropylamine de formule IIa

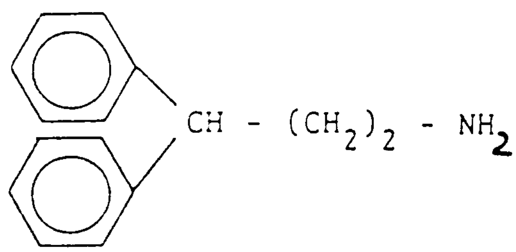

(IIa),

avec une cétone de formule générale IIIa

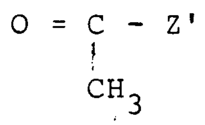

(IIIa)

dans laquelle Z' représente le diphényl-4-yle, le 3,4-diméthylphényle ou le 2,4-diméthylphényle, et, si désiré, transformation du composé obtenu avec des acides minéraux ou organiques en un sel d'addition d'acide correspondant.

8. Produits pharmaceutiques caractérisés en ce qu'ils contiennent un ou plusieurs composés selon les revendications 1 à 5 et/ou leurs sels d'addition avec des acides physiologiquement compatibles, comme substances actives, associés à des adjuvants et/ou supports inertes, usuels.

9. Utilisation des composés selon les revendications 1 à 5 pour la préparation de produits pharmaceutiques destinés au traitement des affections cardiaques et des maladies circulatoires.